(19) Europäisches Patentamt / European Patent Office / Office européen des brevets

(11) **EP 3 815 621 B1**

(12) **EUROPEAN PATENT SPECIFICATION**

(45) Date of publication and mention
of the grant of the patent:
**21.12.2022  Bulletin 2022/51**

(21) Application number: **19847281.3**

(22) Date of filing: **29.05.2019**

(51) International Patent Classification (IPC):
**A61B 10/00** (2006.01)     **A61B 3/113** (2006.01)
**A61B 5/16** (2006.01)      **G16H 50/20** (2018.01)
**G09B 7/00** (2006.01)

(52) Cooperative Patent Classification (CPC):
**G16H 50/20; A61B 5/163; A61B 5/4088;
A61B 5/7282; A61B 5/743; G09B 7/06;
G09B 19/00; G09B 21/008**

(86) International application number:
**PCT/JP2019/021401**

(87) International publication number:
**WO 2020/031471 (13.02.2020 Gazette 2020/07)**

(54) **ASSESSMENT DEVICE, ASSESSMENT METHOD, AND ASSESSMENT PROGRAM**

BEWERTUNGSVORRICHTUNG, BEWERTUNGSVERFAHREN UND BEWERTUNGSPROGRAMM

DISPOSITIF D'ÉVALUATION, PROCÉDÉ D'ÉVALUATION ET PROGRAMME D'ÉVALUATION

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **08.08.2018   JP 2018149559
29.01.2019   JP 2019013002**

(43) Date of publication of application:
**05.05.2021   Bulletin 2021/18**

(73) Proprietor: **JVCKENWOOD Corporation
Yokohama-shi, Kanagawa 221-0022 (JP)**

(72) Inventor: **SHUDO, Katsuyuki
Yokohama-shi, Kanagawa 221-0022 (JP)**

(74) Representative: **Emde, Eric
Wagner & Geyer Partnerschaft mbB
Patent- und Rechtsanwälte
Gewürzmühlstrasse 5
80538 München (DE)**

(56) References cited:
WO-A1-2014/208761     WO-A1-2016/052646
WO-A1-2018/216347     WO-A1-2019/098173
JP-A- 2003 038 443     JP-A- 2003 038 443
JP-A- 2014 068 932     JP-A- 2017 158 866
JP-A- 2018 140 007     US-A1- 2010 092 929
US-A1- 2010 092 929

**Description**

Field

The present invention relates to an evaluation apparatus, an evaluation method, and an evaluation program.
Background

In recent years, cognitive impairment and brain impairment tend to increase, and it is demanded to detect cognitive impairment and brain impairment early and to quantitatively evaluate severity of symptoms. It is known that symptoms of cognitive impairment and brain impairment affect cognitive ability. Therefore, it is common to evaluate a subject based on cognitive ability of the subject. For example, an apparatus that displays a plurality of kinds of numbers, instructs a subject to add the numbers to obtain an answer, and checks the answer provided by the subject has been proposed (for example, see JP 2011-083403 A).

US2010092929A1 discloses methods for assessing cognitive and linguistic abilities by tracking and recording the eye movements of a patient in response to predetermined verbal and visual stimuli. The methods incorporate conventional eye-tracking technology to acquire eye-fixation location and duration measures for testing linguistic comprehension, working memory, attention allocation, and the effect of semantic associative priming. Visual stimuli presented in the methods are carefully designed to reduce visually distracting features. Verbal stimuli are carefully designed to control for numerous linguistic features. JP2003038443A discloses a multivariate computing means which selects a discrimination value from various indexes by a discriminant analysis method in multivariate analysis, and the function thereof which is as follows. The computing means is formed by a multivariate analysis calculating part for calculating a discrimination value w1 of a subject by using an index for evaluating the motion of an eyeball M of the subject obtained by an eyeball motion examining means and an index related to a neuropsychologic examination obtained by a neuropsychologic examination means and converting the same to smaller indexes, and a multivariate analysis calculating part for calling a number of subject indexes obtained by the eyeball motion examining means and the neuropsychologic examination means and stored in a data base and performing multivariate analysis calculation to discrimination values of a number of subjects, and the discrimination values are displayed by an output means.

Summary

Patent Literature 1: Japanese Laid-open Patent Publication No. 2011-083403 A

Technical Problem

However, in the method of Patent Literature 1 or the like, the subject selects an answer by operating a touch panel or the like, so that it is difficult to perform verification including contingency and it is difficult to ensure high evaluation accuracy. Therefore, there is a need to evaluate cognitive impairment and brain impairment with high accuracy.

The present invention has been conceived in view of the foregoing situation, and an object of the present invention is to provide an evaluation apparatus, an evaluation method, and an evaluation program capable of evaluating cognitive impairment and brain impairment with high accuracy.

Solution to Problem

In accordance with the present invention, an evaluation apparatus, method and a program as set forth in the appended claims is provided. In particular, , an evaluation apparatus according to the present invention comprising: a display screen; a gaze point detection unit that detects a position of a gaze point of a subject who observes the display screen; a display control unit that performs display operation including first display operation of displaying question information that is a question for the subject on the display screen, second display operation of displaying a guidance target object that guides the gaze point of the subject to a target position on the display screen, and third display operation of displaying, at positions that do not overlap with the guidance position on the display screen, a plurality of answer target objects that are answers for the question on the display screen after the second display operation; a region setting unit that sets a specific region corresponding to a specific target object among the plurality of the answer target objects and comparison regions corresponding to comparison target objects that are different from the specific target object; a determination unit that determines whether the gaze point is present in the specific region and the comparison region during the display period in which the third display operation is performed, on the basis of the position of the gaze point; an arithmetic unit that calculates gaze point data during the display period on the basis of a determination result of the determination unit; and an evaluation unit that obtains evaluation data of the subject on the basis of the gaze point data, wherein the determination unit (218) is configured to detect whether the gaze point is present in a predetermined region including the target position after the second display operation on the basis of the position of the gaze point, and performs the determination if the gaze point is present, or repeats processes from the first display operation if the gaze point is not present.

An evaluation method according to the present invention comprising: displaying an image on a display screen; detecting a position of a gaze point of a subject who observes the display screen; performing display operation including first display operation of displaying question information that is a question for the subject on the display screen, second display operation of displaying a guidance target object that guides the gaze point of the subject to a target position on the display screen, and third display operation of displaying, at positions that do not overlap with the guidance position on the display screen, a plurality of answer target objects that are answers for the question on the display screen after the second display operation; setting a specific region corresponding to a specific target object among the plurality of the answer target objects and comparison regions corresponding to comparison target objects that are different from the specific target object; determining whether the gaze point is present in the specific region and the comparison region during the display period in which the third display operation is performed, on the basis of the position of the gaze point; calculating gaze point data during the display period on the basis of a determination result of the determination unit; and obtaining evaluation data of the subject on the basis of the gaze point data, wherein the determination unit (218) is configured to detect whether the gaze point is present in a predetermined region including the target position after the second display operation on the basis of the position of the gaze point, and performs the determination if the gaze point is present, or repeats processes from the first display operation if the gaze point is not present.

An evaluation program according to the present invention that causes a computer to execute: a process of displaying an image on a display screen; a process of detecting a position of a gaze point of a subject who observes the display screen; a process of performing display operation including first display operation of displaying question information that is a question for the subject on the display screen, second display operation of displaying a guidance target object that guides the gaze point of the subject to a target position on the display screen, and third display operation of displaying, at positions that do not overlap with the guidance position on the display screen, a plurality of answer target objects that are answers for the question on the display screen after the second display operation; a process of setting a specific region corresponding to a specific target object among the plurality of the answer target objects and comparison regions corresponding to comparison target objects that are different from the specific target object;

a process of determining whether the gaze point is present in the specific region and the comparison region during the display period in which the third display operation is performed, on the basis of the position of the gaze point; a process of calculating gaze point data during the display period on the basis of a determination result of the determination unit; and a process of obtaining evaluation data of the subject on the basis of the gaze point data, wherein the determination unit (218) is configured to detect whether the gaze point is present in a predetermined region including the target position after the second display operation on the basis of the position of the gaze point, and performs the determination if the gaze point is present, or repeats processes from the first display operation if the gaze point is not present.

Advantageous Effects of Invention

According to the present invention, it is possible to provide an evaluation apparatus, an evaluation method, and an evaluation program capable of evaluating cognitive impairment and brain impairment with high accuracy.

Brief Description of Drawings

FIG. 1 is a perspective view schematically illustrating an example of a line-of-sight detection apparatus according to a present embodiment.
FIG. 2 is a diagram illustrating an example of a hardware configuration of the line-of-sight detection apparatus according to the present embodiment.
FIG. 3 is a functional block diagram illustrating an example of the line-of-sight detection apparatus according to the present embodiment.
FIG. 4 is a schematic diagram for explaining a method of calculating positional data of a corneal curvature center according to the present embodiment.
FIG. 5 is a schematic diagram for explaining the method of calculating the positional data of the corneal curvature center according to the present embodiment.
FIG. 6 is a schematic diagram for explaining an example of a calibration process according to the present embodiment.
FIG. 7 is a schematic diagram for explaining an example of a gaze point detection process according to the present embodiment.

FIG. 8 is a diagram illustrating an example of question information displayed on a display screen.

FIG. 9 is a diagram illustrating an example of a guidance target object displayed on the display screen.

FIG. 10 is a diagram illustrating an example of answer target objects displayed on the display screen.

FIG. 11 is a diagram illustrating an example of regions set on the display screen.

FIG. 12 is a diagram illustrating an example of question information displayed on the display screen.

FIG. 13 is a diagram illustrating an example of a guidance target object displayed on the display screen.

FIG. 14 is a diagram illustrating an example of answer target objects displayed on the display screen.

FIG. 15 is a diagram illustrating an example of regions set on the display screen.

FIG. 16 is a diagram illustrating another display example of the guidance target object displayed on the display screen.

FIG. 17 is a diagram illustrating another display example of answer target objects displayed on the display screen.

FIG. 18 is a diagram illustrating an example of instruction information displayed on the display screen.

FIG. 19 is a diagram illustrating an example of question information displayed on the display screen.

FIG. 20 is a diagram illustrating an example of a guidance target object displayed on the display screen.

FIG. 21 is a diagram illustrating an example of answer target objects displayed on the display screen.

FIG. 22 is a diagram illustrating another display example of answer target objects displayed on the display screen.

FIG. 23 is a flowchart illustrating an example of an evaluation method according to the present embodiment.

FIG. 24 is a diagram illustrating an example of operation that is performed after second display operation is performed.

FIG. 25 is a flowchart illustrating another example of the evaluation method according to the present embodiment.

FIG. 26 is a diagram illustrating another example of the operation that is performed after the second display operation is performed.

FIG. 27 is a diagram illustrating an example of the answer target objects displayed on the display screen.

FIG. 28 is a flowchart illustrating still another example of the evaluation method according to the present embodiment.

FIG. 29 is a diagram illustrating another example of question information displayed on the display screen.

FIG. 30 is a diagram illustrating still another example of question information displayed on the display screen.

FIG. 31 is a diagram illustrating still another example of question information displayed on the display screen.

FIG. 32 is a diagram illustrating another example of a guidance target object displayed on the display screen.

FIG. 33 is a diagram illustrating another example of answer target objects displayed on the display screen.

FIG. 34 is a flowchart illustrating another example of a process in first display operation.

FIG. 35 is a flowchart illustrating another example of a process in the first display operation and the second display operation.

FIG. 36 is a diagram illustrating another example of instruction information displayed on the display screen.

FIG. 37 is a diagram illustrating still another example of question information displayed on the display screen.

FIG. 38 is a diagram illustrating still another example of a guidance target object displayed on the display screen.

FIG. 39 is a diagram illustrating still another example of question information displayed on the display screen.

FIG. 40 is a diagram illustrating still another example of question information displayed on the display screen.

FIG. 41 is a diagram illustrating still another example of question information displayed on the display screen.

FIG. 42 is a diagram illustrating still another example of question information displayed on the display screen.

Description of Embodiments

Embodiments of an evaluation apparatus, an evaluation method, and an evaluation program according to the present invention will be described below based on the drawings. The present invention is not limited by the embodiments below. In addition, constituent elements described in the embodiments below include one that can be easily replaced by a person skilled in the art and one that is practically identical.

In the description below, a three-dimensional global coordinate system is set to describe positional relationships among components. A direction parallel to a first axis of a predetermined plane is referred to as an X-axis direction, a direction parallel to a second axis perpendicular to the first axis in the predetermined plane is referred to as a Y-axis direction, and a direction perpendicular to each of the first axis and the second axis is referred to a Z-axis direction. The predetermined plane includes an XY plane.

Line-of-sight Detection Apparatus

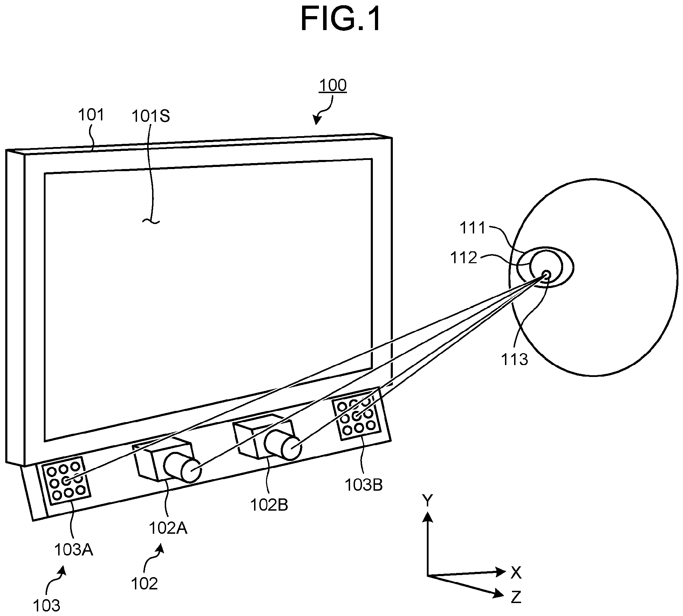

FIG. 1 is a perspective view schematically illustrating an example of a line-of-sight detection apparatus 100 according to a first embodiment. The line-of-sight detection apparatus 100 is used as an evaluation apparatus that evaluates cognitive impairment and brain impairment. As illustrated in FIG. 1, the line-of-sight detection apparatus 100 includes a display device 101, a stereo camera device 102, and a lighting device 103.

The display device 101 includes a flat panel display, such as a liquid crystal display (LCD) or an organic electroluminescence (EL) display (OLED). In the present embodiment, the display device 101 includes a display screen 101S. The display screen 101S displays an image. In the present embodiment, the display screen 101S displays, for example, an index for evaluating visual performance of a subject. The display screen 101S is substantially parallel to the XY plane. The X-axis direction corresponds to a horizontal direction of the display screen 101S, the Y-axis direction corresponds to a vertical direction of the display screen 101S, and the Z-axis direction corresponds to a depth direction perpendicular to the display screen 101S.

The stereo camera device 102 includes a first camera 102A and a second camera 102B. The stereo camera device 102 is arranged below the display screen 101S of the display device 101. The first camera 102A and the second camera 102B are arranged in the X-axis direction. The first camera 102A is arranged in the negative X direction relative to the second camera 102B. Each of the first camera 102A and the second camera 102B includes an infrared camera, an optical system capable of transmitting near-infrared light with a wavelength of 850 nanometers (nm) for example, and an imaging element capable of receiving the near-infrared light.

The lighting device 103 includes a first light source 103A and a second light source 103B. The lighting device 103 is arranged below the display screen 101S of the display device 101. The first light source 103A and the second light source 103B are arranged in the X-axis direction. The first light source 103A is arranged in the negative X direction relative to the first camera 102A. The second light source 103B is arranged in the positive X direction relative to the second camera 102B. Each of the first light source 103A and the second light source 103B includes a light emitting diode (LED) light source and is able to emit near-infrared light with a wavelength of 850 nm, for example. Meanwhile, the first light source 103A and the second light source 103B may be arranged between the first camera 102A and the second camera 102B.

The lighting device 103 emits near-infrared light as detection light and illuminates an eyeball 111 of the subject. The stereo camera device 102 captures an image of a part of the eyeball 111 (hereinafter, the part of the eyeball is also be referred to as the "eyeball") by the second camera 102B when the eyeball 111 is irradiated with the detection light emitted from the first light source 103A, and captures an image of the eyeball 111 by the first camera 102A when the eyeball 111 is irradiated with the detection light emitted from the second light source 103B.

At least one of the first camera 102A and the second camera 102B outputs a frame synchronous signal. The first light source 103A and the second light source 103B output detection light based on the frame synchronous signal. The first camera 102A captures image data of the eyeball 111 when the eyeball 111 is irradiated with the detection light emitted from the second light source 103B. The second camera 102B captures image data of the eyeball 111 when the eyeball 111 is irradiated with the detection light emitted from the first light source 103A.

If the eyeball 111 is irradiated with the detection light, a part of the detection light is reflected by a pupil 112, and light from the pupil 112 enters the stereo camera device 102. Further, if the eyeball 111 is irradiated with the detection light, a corneal reflection image 113 that is a virtual image of a cornea is formed on the eyeball 111, and light from the corneal reflection image 113 enters the stereo camera device 102.

By appropriately setting the relative position between the first camera 102A/the second camera 102B and the first light source 103A/the second light source 103B, intensity of light that enters from the pupil 112 to the stereo camera device 102 is reduced, and intensity of light that enters from the corneal reflection image 113 to the stereo camera device 102 is increased. In other words, an image of the pupil 112 captured by the stereo camera device 102 has low luminance, and an image of the corneal reflection image 113 has high luminance. The stereo camera device 102 is able to detect a position of the pupil 112 and a position of the corneal reflection image 113 on the basis of the luminance of the captured image.

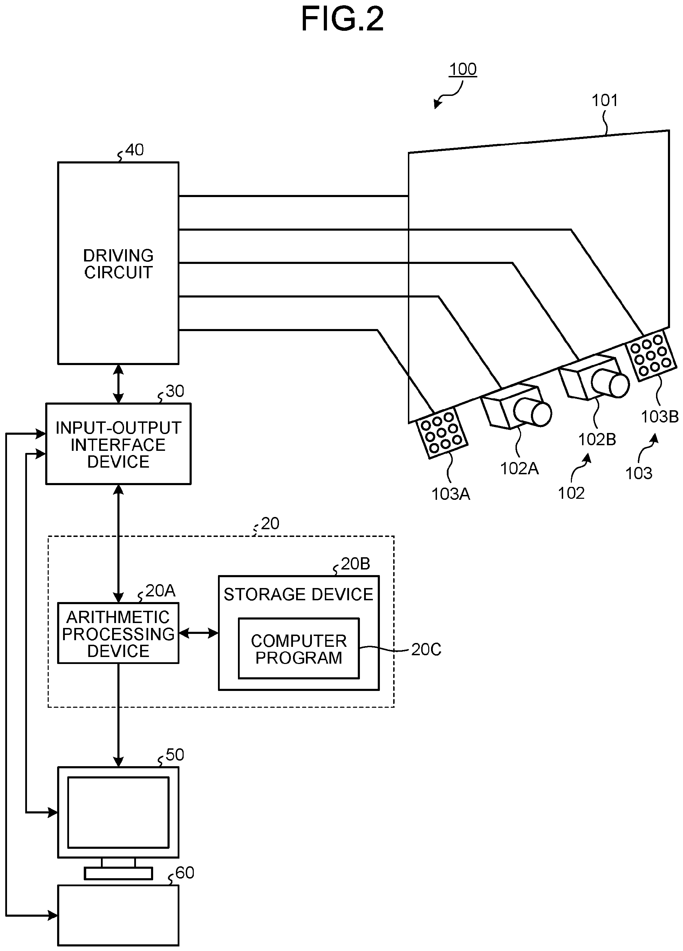

FIG. 2 is a diagram illustrating an example of a hardware configuration of the line-of-sight detection apparatus 100 according to the present embodiment. As illustrated in FIG. 2, the line-of-sight detection apparatus 100 includes the display device 101, the stereo camera device 102, the lighting device 103, a computer system 20, an input-output interface device 30, a driving circuit 40, an output device 50, and an input device 60.

The computer system 20, the driving circuit 40, the output device 50, and the input device 60 perform data communication via the input-output interface device 30. The computer system 20 includes an arithmetic processing device 20A and a storage device 20B. The arithmetic processing device 20A includes a microprocessor, such as a central processing unit (CPU). The storage device 20B includes a memory, such as a read only memory (ROM) and a random access memory (RAM), or a storage. The arithmetic processing device 20A performs an arithmetic process in accordance with a computer program 20C that is stored in the storage device 20B.

The driving circuit 40 generates a driving signal and outputs the driving signal to the display device 101, the stereo camera device 102, and the lighting device 103. Further, the driving circuit 40 supplies image data of the eyeball 111 that is captured by the stereo camera device 102 to the computer system 20 via the input-output interface device 30.

The output device 50 includes a display device, such as a flat panel display. The output device 50 may include a printing device. The input device 60 generates input data by being operated. The input device 60 includes a keyboard or a mouse for a computer system. The input device 60 may include a touch sensor that is arranged on a display screen of the output device 50 that serves as a display device.

In the present embodiment, the display device 101 and the computer system 20 are separated devices. However, the display device 101 and the computer system 20 may be integrated. For example, if the line-of-sight detection apparatus 100 includes a tablet personal computer, the computer system 20, the input-output interface device 30, the driving circuit 40, and the display device 101 may be mounted on the tablet personal computer.

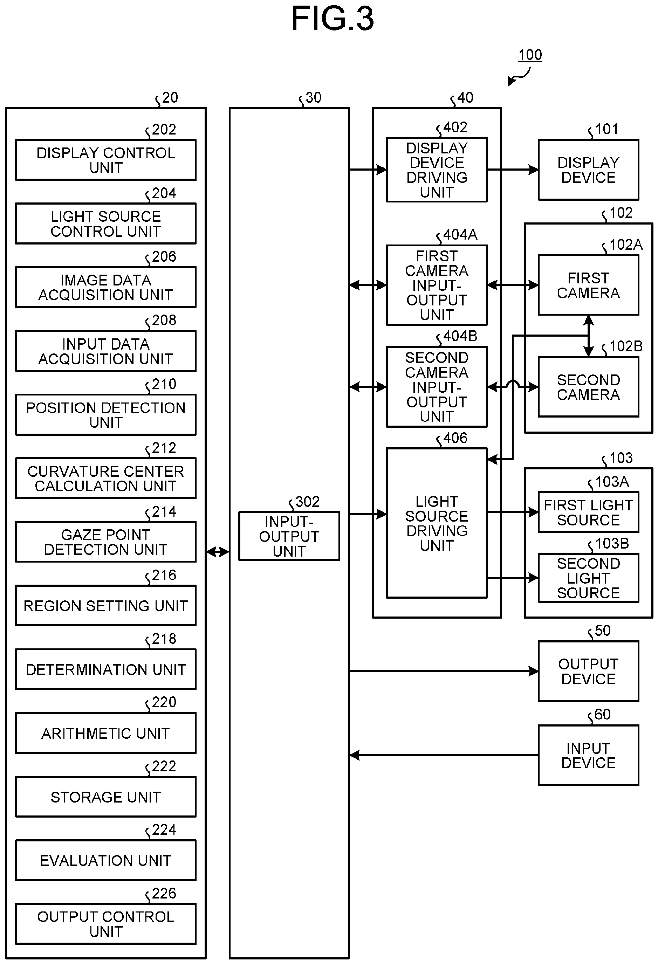

FIG. 3 is a functional block diagram illustrating an example of the line-of-sight detection apparatus 100 according to the present embodiment. As illustrated in FIG. 3, the input-output interface device 30 includes an input-output unit 302. The driving circuit 40 includes a display device driving unit 402 that generates a driving signal for driving the display device 101 and outputs the driving signal to the display device 101, a first camera input-output unit 404A that generates a driving signal for driving the first camera 102A and outputs the driving signal to the first camera 102A, a second camera input-output unit 404B that generates a driving signal for driving the second camera 102B and outputs the driving signal to the second camera 102B, and a light source driving unit 406 that generates a driving signal for driving the first light source 103A and the second light source 103B and outputs the driving signal to the first light source 103A and the second light source 103B. Further, the first camera input-output unit 404A supplies image data of the eyeball 111 that is captured by the first camera 102A to the computer system 20 via the input-output unit 302. The second camera input-output unit 404B supplies image data of the eyeball 111 that is captured by the second camera 102B to the computer system 20 via the input-output unit 302.

The computer system 20 controls the line-of-sight detection apparatus 100. The computer system 20 includes a display control unit 202, a light source control unit 204, an image data acquisition unit 206, an input data acquisition unit 208, a position detection unit 210, a curvature center calculation unit 212, a gaze point detection unit 214, a region setting unit 216, a determination unit 218, an arithmetic unit 220, a storage unit 222, an evaluation unit 224, and an output control unit 226. Functions of the computer system 20 are implemented by the arithmetic processing device 20A and the storage device 20B.

The display control unit 202 performs display operation including first display operation of displaying question information that is a question for the subject on the display screen 101S, second display operation of displaying a guidance target object that guides a gaze point of the subject to a target position on the display screen, and third display operation of displaying a plurality of answer target objects that are answers for the question at positions that do not overlap with a guidance position on the display screen 101S after the second display operation. The question information includes characters, figures, and the like. The guidance target object includes an eye-catching video or the like that guides the gaze point to a desired position on the display screen 101S. The eye-catching video allows the subject to start viewing from a target position of an evaluation image. The target position may be set at a certain position that is desired to be gazed at by the subject in the evaluation image at the start of display of the evaluation image. The plurality of answer target objects include, for example, a specific target object that is a correct answer for the question and comparison target objects that are different from the specific target object. The question information, the guidance target object, and the answer target objects as described above are included in, for example, an evaluation video or an evaluation image that is to be viewed by the subject. The display control unit 202 displays the evaluation video or the evaluation image as described above on the display screen 101S.

The light source control unit 204 controls the light source driving unit 406, and controls operation states of the first light source 103A and the second light source 103B. The light source control unit 204 controls the first light source 103A and the second light source 103B such that the first light source 103A and the second light source 103B emit detection light at different timings.

The image data acquisition unit 206 acquires the image data of the eyeball 111 of the subject that is captured by the stereo camera device 102 including the first camera 102A and the second camera 102B, from the stereo camera device 102 via the input-output unit 302.

The input data acquisition unit 208 acquires the input data that is generated through operation of the input device 60, from the input device 60 via the input-output unit 302.

The position detection unit 210 detects positional data of a pupil center on the basis of the image data of the eyeball 111 acquired by the image data acquisition unit 206. Further, the position detection unit 210 detects positional data of a corneal reflection center on the basis of the image data of the eyeball 111 acquired by the image data acquisition unit 206. The pupil center is a center of the pupil 112. The corneal reflection center is a center of the corneal reflection image 113. The position detection unit 210 detects the positional data of the pupil center and the positional data of the corneal reflection center for each of the right and left eyeballs 111 of the subject.

The curvature center calculation unit 212 calculates positional data of a corneal curvature center of the eyeball 111 on the basis of the image data of the eyeball 111 acquired by the image data acquisition unit 206.

The gaze point detection unit 214 detects positional data of a gaze point of the subject on the basis of the image data of the eyeball 111 acquired by the image data acquisition unit 206. In the present embodiment, the positional data of the gaze point indicates positional data of an intersection point between a line-of-sight vector of the subject that is defined by the three-dimensional global coordinate system and the display screen 101S of the display device 101. The gaze point detection unit 214 detects a line-of-sight vector of each of the right and left eyeballs 111 of the subject on the basis of the positional data of the pupil center and the positional data of the corneal curvature center that are acquired from the image data of the eyeball 111. After detection of the line-of-sight vector, the gaze point detection unit 214 detects the positional data of the gaze point that indicates the intersection point between the line-of-sight vector and the display screen 101S.

The region setting unit 216 sets a specific region corresponding to the specific target object and comparison regions corresponding to the respective comparison target objects on the display screen 101S of the display device 101 during a display period in which the third display operation is performed.

The determination unit 218 determines, on the basis of positional data of a viewpoint, whether the gaze point is present in each of the specific region and the comparison regions during the display period in which the third display operation is performed, and outputs determination data. The determination unit 218 determines whether the gaze point is present in the specific region and the comparison regions at a constant time interval, for example. The constant time interval may be set to, for example, a cycle of the frame synchronous signal (for example, every 20 milliseconds (msec)) that is output from the first camera 102A and the second camera 102B.

The arithmetic unit 220 calculates movement course data (may be described as gaze point data) that indicates a course of movement of the gaze point during the display period, on the basis of the determination data of the determination unit 218. The movement course data includes arrival time data indicating a time period from a start time of the display period to an arrival time at which the gaze point first arrives at the specific region, movement frequency data indicating the number of times of movement of the position of the gaze point among the plurality of comparison regions before the gaze point first arrives at the specific region, presence time data indicating a presence time in which the gaze point is present in the specific region or the comparison regions during the display period, and final region data indicating a region in which the gaze point is finally located among the specific region and the comparison regions during a display time.

Meanwhile, the arithmetic unit 220 includes a management timer for managing a video replay time, and a detection timer T for detecting an elapsed time since start of display of the video on the display screen 101S. The arithmetic unit 220 includes a counter that counts the number of times the gaze point is determined as being present in the specific region.

The evaluation unit 224 obtains evaluation data of the subject on the basis of the movement course data. The evaluation data is data for evaluating whether the subject is able to gaze at the specific target object that is displayed on the display screen 101S in the display operation.

The storage unit 222 stores therein the determination data, the movement course data (the presence time data, the movement frequency data, the final region data, and the arrival time data), and the evaluation data as described above. Further, the storage unit 222 stores therein an evaluation program that causes a computer to execute a process of displaying an image on the display screen, a process of detecting the position of the gaze point of the subject who observes the display screen, a process of performing the display operation including the first display operation of displaying the question information that is a question for the subject on the display screen, the second display operation of displaying the guidance target object that guides the gaze point of the subject to the target position on the display screen, and the third display operation of displaying the plurality of answer target objects that are answers for the question at positions that do not overlap with the guidance position, a process of setting the specific region corresponding to the specific target object among the plurality of answer target objects and the comparison target objects different from the specific target object, a process of determining, on the basis of the position of the gaze point, whether the gaze point is present in the specific region and the comparison regions during the display period in which the third display operation is performed, a process of calculating the gaze point data during the display period on the basis of a determination result, and a process of obtaining the evaluation of the subject on the basis of the gaze point data.

The output control unit 226 outputs data to at least one of the display device 101 and the output device 50.

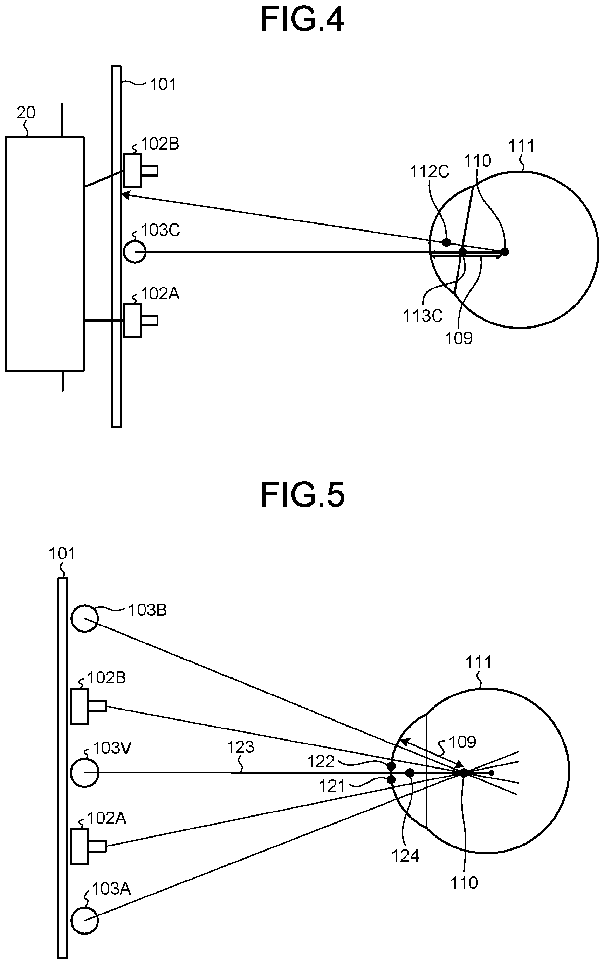

An overview of a process performed by the curvature center calculation unit 212 according to the present embodiment will be described below. The curvature center calculation unit 212 calculates the positional data of the corneal curvature center of the eyeball 111 on the basis of the image data of the eyeball 111. FIG. 4 and FIG. 5 are schematic diagrams for explaining a method of calculating positional data of a corneal curvature center 110 according to the present embodiment. FIG. 4 illustrates an example in which the eyeball 111 is illuminated by a single light source 103C. FIG. 5 illustrates an example in which the eyeball 111 is illuminated by the first light source 103A and the second light source 103B.

First, the example illustrated in FIG. 4 will be explained. The light source 103C is arranged between the first camera 102A and the second camera 102B. A pupil center 112C is the center of the pupil 112. A corneal reflection center 113C is the center of the corneal reflection image 113. In FIG. 4, the pupil center 112C indicates a pupil center that is obtained when the eyeball 111 is illuminated by the single light source 103C. The corneal reflection center 113C indicates a corneal reflection center that is obtained when the eyeball 111 is illuminated by the single light source 103C. The corneal reflection center 113C is located on a straight line that connects the light source 103C and the corneal curvature center 110. The corneal reflection center 113C is located at an intermediate point between a corneal surface and the corneal curvature center 110. A corneal curvature radius 109 is a distance between the corneal surface and the corneal curvature center 110. Positional data of the corneal reflection center 113C is detected by the stereo camera device 102. The corneal curvature center 110 is located on a straight line that connects the light source 103C and the corneal reflection center 113C. The curvature center calculation unit 212 calculates, as the positional data of the corneal curvature center 110, positional data for which a distance from the corneal reflection center 113C on the straight line is equal to a predetermined value. The predetermined value is a value that is determined in advance from a curvature radius value of a general cornea or the like, and stored in the storage unit 222.

Next, the example illustrated in FIG. 5 will be described. In the present embodiment, the first camera 102A/the second light source 103B are arranged at bilaterally symmetrical positions and the second camera 102B/the first light source 103A are arranged at bilaterally symmetrical positions with respect to a straight line that passes through an intermediate position between the first camera 102A and the second camera 102B. It is assumed that a virtual light source 103V is present at the intermediate position between the first camera 102A and the second camera 102B. A corneal reflection center 121 indicates a corneal reflection center in an image that is obtained by capturing the eyeball 111 by the second camera 102B. A corneal reflection center 122 indicates a corneal reflection center in an image that is obtained by capturing the eyeball 111 by the first camera 102A. A corneal reflection center 124 indicates a corneal reflection center corresponding to the virtual light source 103V. Positional data of the corneal reflection center 124 is calculated based on the positional data of the corneal reflection center 121 and the positional data of the corneal reflection center 122 that are captured by the stereo camera device 102. The stereo camera device 102 detects the positional data of the corneal reflection center 121 and the positional data of the corneal reflection center 122 in a three-dimensional local coordinate system that is defined in the stereo camera device 102. Camera calibration using a stereo calibration method is performed in advance on the stereo camera device 102, and a transformation parameter for transforming the three-dimensional local coordinate system of the stereo camera device 102 into the three-dimensional global coordinate system is calculated. The transformation parameter is stored in the storage unit 222. The curvature center calculation unit 212 transforms the positional data of the corneal reflection center 121 and the positional data of the corneal reflection center 122, which are captured by the stereo camera device 102, into pieces of positional data in the three-dimensional global coordinate system by using the transformation parameter. The curvature center calculation unit 212 calculates the positional data of the corneal reflection center 124 in the three-dimensional global coordinate system, on the basis of the positional data of the corneal reflection center 121 and the positional data of the corneal reflection center 122 that are defined in the three-dimensional global coordinate system. The corneal curvature center 110 is located on a straight line that connects the virtual light source 103V and the corneal reflection center 124. The curvature center calculation unit 212 calculates, as the positional data of the corneal curvature center 110, positional data for which a distance from the corneal reflection center 124 on a straight line 123 is equal to a predetermined value. The predetermined value is a value that is determined in advance from a curvature radius value of a general cornea or the like, and stored in the storage unit 222.

As described above, even if the two light sources are provided, the corneal curvature center 110 is calculated by the same method as the method that is adopted when the single light source is provided.

The corneal curvature radius 109 is a distance between the corneal surface and the corneal curvature center 110. Therefore, the corneal curvature radius 109 is calculated by calculating positional data of the corneal surface and the positional data of the corneal curvature center 110.

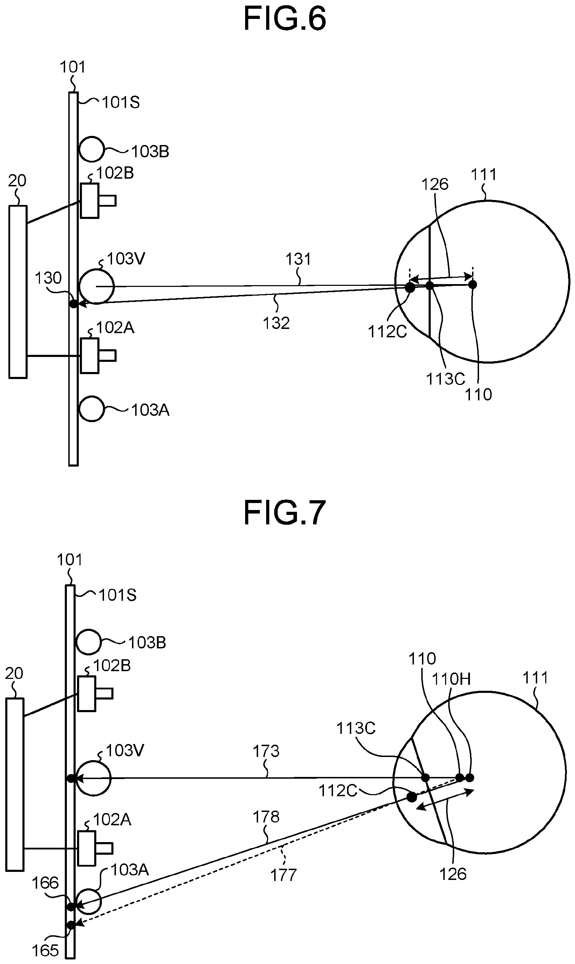

Next, an example of the line-of-sight detection method according to the present embodiment will be described. FIG. 6 is a schematic diagram for explaining an example of a calibration process according to the present embodiment. In the calibration process, a target position 130 is set so as to be gazed at by the subject. The target position 130 is defined in the three-dimensional global coordinate system. In the present embodiment, the target position 130 is set at a central position of the display screen 101S of the display device 101, for example. Meanwhile, the target position 130 may be set at a position of an end portion of the display screen 101S. The output control unit 226 displays a target image at the set target position 130. A straight line 131 is a straight line that connects the virtual light source 103V and the corneal reflection center 113C. A straight line 132 is a straight line that connects the target position 130 and the pupil center 112C. The corneal curvature center 110 is an intersection point between the straight line 131 and the straight line 132. The curvature center calculation unit 212 is able to calculate the positional data of the corneal curvature center 110 on the basis of positional data of the virtual light source 103V, positional data of the target position 130, positional data of the pupil center 112C, and the positional data of the corneal reflection center 113C.

A gaze point detection process will be described below. The gaze point detection process is performed after the calibration process. The gaze point detection unit 214 calculates a line-of-sight vector of the subject and the positional data of the gaze point on the basis of the image data of the eyeball 111. FIG. 7 is a schematic diagram for explaining an example of the gaze point detection process according to the present embodiment. In FIG. 7, a gaze point 165 indicates a gaze point that is obtained from a corneal curvature center that is calculated using a general curvature radius value. A gaze point 166 indicates a gaze point that is obtained from a corneal curvature center that is calculated using a distance 126 obtained in the calibration process. The pupil center 112C indicates the pupil center that is calculated in the calibration process, and the corneal reflection center 113C indicates the corneal reflection center that is calculated in the calibration process. A straight line 173 is a straight line that connects the virtual light source 103V and the corneal reflection center 113C. The corneal curvature center 110 is a position of the corneal curvature center that is calculated from a general curvature radius value. The distance 126 is a distance between the pupil center 112C that is calculated in the calibration process and the corneal curvature center 110. A corneal curvature center 110H indicates a position of a corrected corneal curvature center that is obtained by correcting the corneal curvature center 110 using the distance 126. The corneal curvature center 110H is obtained under the condition that the corneal curvature center 110 is located on the straight line 173 and the distance between the pupil center 112C and the corneal curvature center 110 is the distance 126. Accordingly, a line of sight 177 that is calculated using a general curvature radius value is corrected to a line of sight 178. Further, the gaze point on the display screen 101S of the display device 101 is corrected from the gaze point 165 to the gaze point 166.

Evaluation Method

The evaluation method according to the present embodiment will be described below. In the evaluation method according to the present embodiment, cognitive impairment and brain impairment of the subject are evaluated by using the line-of-sight detection apparatus 100 as described above.

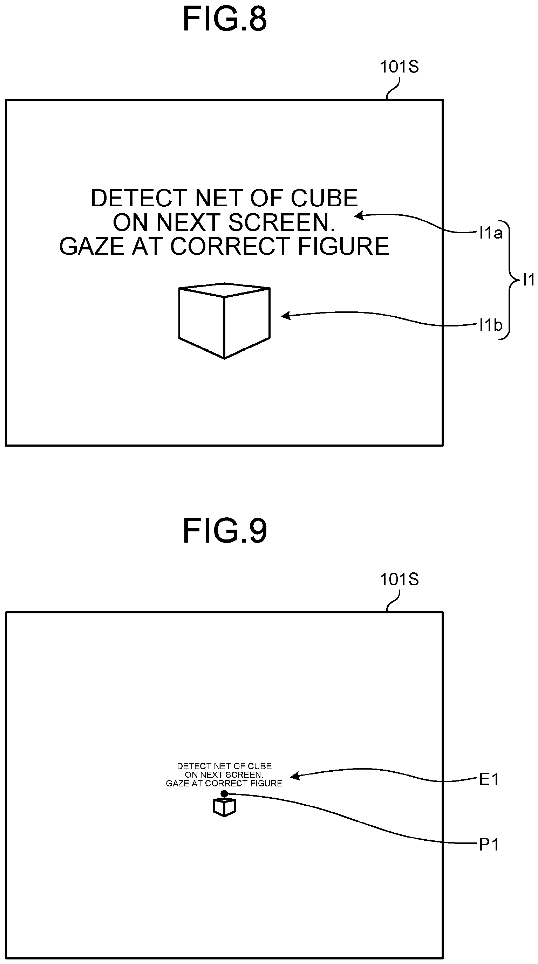

FIG. 8 is a diagram illustrating an example of question information I1 that is displayed on the display screen 101S in the evaluation method according to the present embodiment. As illustrated in FIG. 8, the display control unit 202 displays, as the first display operation, the question information I1 that is a question for the subject on the display screen 101S. In the present embodiment, the question information I1 is a question indicating an instruction to gaze at a figure that is correct for a net of a cube. The display control unit 202 displays, as the question information I1, character information I1a, such as a sentence, and figure information I1b, such as a figure, but embodiments are not limited thereto, and it may be possible to display only the character information I1a.

After displaying the question information I1 on the display screen 101S, the display control unit 202 displays, as the second display operation, a guidance target object E1 on the display screen 101S. FIG. 9 is a diagram illustrating an example of the guidance target object E1 displayed on the display screen 101S. As illustrated in FIG. 9, in the second display operation, the display control unit 202 displays a video of the guidance target object E1, which is obtained by reducing the above-described question information I1 toward a predetermined target position P1 on the display screen 101S, as an eye-catching video on the display screen 101S. In this example, the target position P1 is set at a position of the center of the display screen 101S, but embodiments are not limited to this example. Meanwhile, while keeping displaying the question information I1 on the display screen 101S, the display control unit 202 may further display, as the guidance target object, a target object that is different from the question information I1 on the display screen 101S and display, as the eye-catching video, a video that is obtained by reducing the target object toward the target position P1 on the display screen 101S.

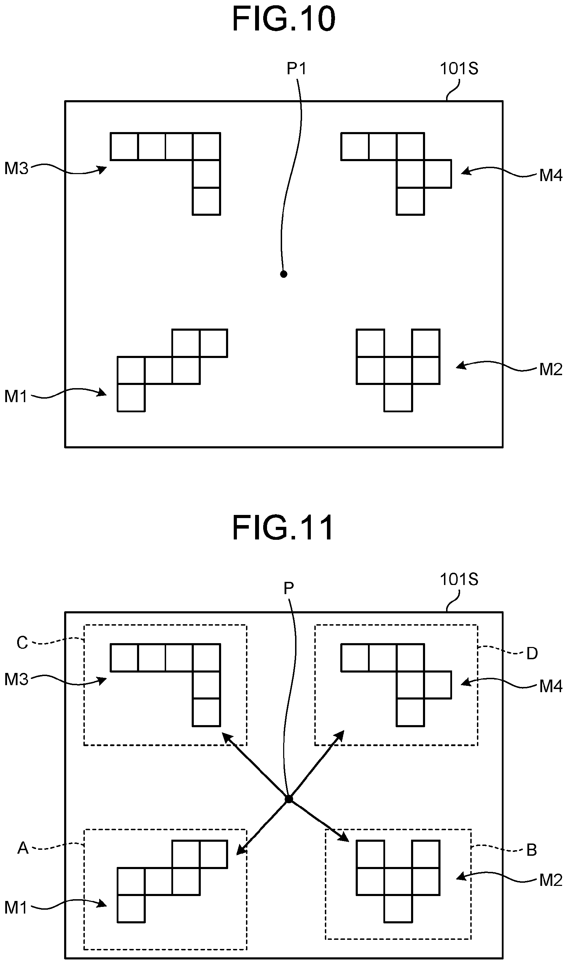

FIG. 10 is a diagram illustrating an example of answer target objects displayed on the display screen 101S. As illustrated in FIG. 10, after performing the second display operation, the display control unit 202 displays, as the third display operation, a plurality of answer target objects M1 to M4 that are certain figures, in each of which six squares are connected, on the display screen 101S. The display control unit 202 displays, as the plurality of answer target objects M1 to M4, the specific target object M1 that is a correct answer for the question information I1 and the comparison target objects M2 to M4 that are different from the specific target object M1 and that are incorrect answers for the question information I1 on the display screen 101S.

The display control unit 202 arranges the plurality of answer target objects M1 to M4 at positions that do not overlap with one another. Further, the display control unit 202 arranges the plurality of answer target objects M1 to M4 at positions that do not overlap with the guidance position. For example, the display control unit 202 arranges the plurality of answer target objects M1 to M4 around the guidance position. In the present embodiment, the guidance position is the target position P1 to which the gaze point of the subject is guided by the guidance target object E1. The display control unit 202 may arrange the plurality of answer target objects M1 to M4 at positions at equal distances from the target position P1 that is the guidance position.

FIG. 11 is a diagram illustrating an example of regions that are set on the display screen 101S during the display period in which the third display operation is performed. As illustrated in FIG. 11, the region setting unit 216 sets a specific region A corresponding to the specific target object M1 during the display period in which the third display operation is performed. Further, the region setting unit 216 sets comparison regions B to D corresponding to the respective comparison target objects M2 to M4. The region setting unit 216 is able to set the specific region A in a region that includes at least a part of the specific target object M1. Further, the region setting unit 216 is able to set the comparison regions B to D in regions including at least respective parts of the comparison target objects M2 to M4. In this case, the region setting unit 216 sets the specific region A and the comparison regions B to D at positions that do not overlap with one another. Meanwhile, the specific region A and the comparison regions B to D are not displayed on the display screen 101S.

Meanwhile, FIG. 11 illustrates one example of a gaze point P that is displayed, as a result, on the display screen 101S after measurement for example, but in reality, the gaze point P is not displayed on the display screen 101S. Positional data of the gaze point is detected with a period of the frame synchronous signal (for example, every 20 msec) that is output from the first camera 102A and the second camera 102B, for example. The first camera 102A and the second camera 102B capture images in a synchronous manner.

The region setting unit 216 sets the specific region A in a rectangular range including the specific target object M1 that is a correct answer for the question information I1. Similarly, the region setting unit 216 sets the comparison regions B to D in respective rectangular ranges including the comparison target objects M2 to M4 that are incorrect answers for the question information I1. Meanwhile, the specific region A and the comparison regions B to D need not always have rectangular shapes, but may have different shapes, such as circles, ellipses, or polygons.

It is known that symptoms of cognitive impairment and brain impairment affect cognitive ability and memory ability of the subject. If the subject does not have cognitive impairment and brain impairment, the subject is able to view, one by one, the comparison target objects M2 to M4 that are displayed on the display screen 101S in the third display operation, determine that it is difficult to assemble a square, and finally detect and gaze at the specific target object M1. Further, if the subject has cognitive impairment and brain impairment, the subject may have difficulty in performing assembly as described above and gazing at the specific target object M1. In contrast, in a method of displaying the answer target object M1 to M4 on the display screen 101S, in some cases, the gaze point of the subject may be accidentally located at the specific target object M1 that is a correct answer at the start of the third display operation. In this case, it may be determined that the answer is correct regardless of whether or not the subject has cognitive impairment and brain impairment, so that it becomes difficult to evaluate the subject with high accuracy.

To cope with this, it is possible to evaluate the subject with high accuracy through the procedure as described below, for example. First, as the first display operation, the question information I1 is displayed on the display screen 101S so as to be checked by the subject. Further, as the second display operation, the guidance target object is displayed on the display screen 101S and the gaze point of the subject is guided to the target position P1. Thereafter, as the third display operation, the plurality of answer target objects M1 to M4 are displayed around the guidance position (the target position P1) on the display screen 101S.

Through the procedure as described above, it is possible to prevent the gaze point of the subject from moving to or being fixed to any of the answer target objects M1 to M4 at the start of the third display operation. Accordingly, it is possible to prevent a situation that is equivalent to a situation in which the subject unintendedly gazes at the answer target object at the start time. Consequently, it is possible to evaluate, with high accuracy, the subject from the standpoint of whether the subject gazes at the plurality of comparison target objects M2 to M4 one by one, whether the subject is able to finally reach the specific target object M1 that is a correct answer, how long does it take before the subject reaches the specific target object M1, and whether the subject is able to gaze at the specific target object M1, for example.

In the third display operation, if the positional data of the gaze point P of the subject is detected, the determination unit 218 determines whether the gaze point of the subject is present in the specific region A and the plurality of comparison regions B to D, and outputs determination data.

The arithmetic unit 220 calculates movement course data that indicates a course of movement of the gaze point P during the display period, on the basis of the determination data. The arithmetic unit 220 calculates, as the movement course data, the presence time data, the movement frequency data, the final region data, and the arrival time data.

The presence time data indicates a presence time in which the gaze point P is present in the specific region A or the comparison regions B to D. In the present embodiment, it is possible to estimate that the presence time in which the gaze point P is present in the specific region A or the comparison regions B to D increases with an increase in the number of times the gaze point is determined as being present in the specific region A or the comparison regions B to D by the determination unit 218. Therefore, it is possible to adopt, as the presence time data, the number of times the gaze point is determined as being present in the specific region A or the comparison regions B to D by the determination unit 218. In other words, the arithmetic unit 220 is able to adopt count values CNTA, CNTB, CNTC, and CNTD of the counter as the presence time data.

Further, the movement frequency data indicates the number of times of movement of the gaze point P among the plurality of comparison regions B to D before the gaze point P first arrives at the specific region A. Therefore, the arithmetic unit 220 is able to count the number of times of movement of the gaze point P among the specific region A and the comparison regions B to D, and adopt, as the movement frequency data, a result of counting that is performed before the gaze point P arrives at the specific region A.

Furthermore, the final region data indicates a region in which the gaze point P is finally located among the specific region A and the comparison regions B to D, that is, a region that is finally gazed at, as the answer, by the subject. The arithmetic unit 220 updates a region in which the gaze point P is present every time the gaze point P is detected, and is accordingly able to adopt a detection result at the end of the display period as the final region data.

Moreover, the arrival time data indicates a time period from the start time of the display period to an arrival time at which the gaze point first arrives at the specific region A. Therefore, by measuring an elapsed time since the start of the display period by the timer T and detecting a measurement value of the timer T by assuming that a flag value is set to 1 at the time the gaze point first arrives at the specific region A, the arithmetic unit 220 is able to adopt a detection result of the timer T as the arrival time data.

In the present embodiment, the evaluation unit 224 obtains evaluation data on the basis of the presence time data, the movement frequency data, the final region data, and the arrival time data.

Here, a data value of the final region data is denoted by D1, a data value of the presence time data of the specific region A is denoted by D2, a data value of the arrival time data is denoted by D3, a data value of the movement frequency data is denoted by D4. However, the data value D1 of the final region data is set to 1 if the final gaze point P of the subject is present in the specific region A (that is, if the answer is correct), and set to 0 if the gaze point P of the subject is not present in the specific region A (that is, if the answer is incorrect). Further, it is assumed that the data value D2 of the presence time data is the number of seconds in which the gaze point P is present in the specific region A. Meanwhile, it may be possible to set, for the data value D2, an upper limit value that is a smaller number of seconds than the display period. Furthermore, the data value D3 of the arrival time data is set to a reciprocal of the arrival time (for example, 1/(arrival time)/10) (10 is a coefficient used to set an arrival time evaluation value to 1 or smaller based on the assumption that a minimum value of the arrival time is 0.1 second). Moreover, the counter value is used as it is as the data value D4 of the movement frequency data. Meanwhile, it may be possible to appropriately set an upper limit value of the data value D4.

In this case, an evaluation value ANS may be represented as follows, for example.

$$ANS = D1 \times K1 + D2 \times K2 + D3 \times K3 + D4 \times K4$$

Meanwhile, K21 to K24 are constants for weighting. The constants K21 to K24 may be set appropriately.

A value of the evaluation value ANS represented by Expression above increases when the data value D1 of the final region data is set to 1, when the data value D2 of the presence time data increases, when the data value D3 of the arrival time data decreases, and when a value of the data value D4 of the movement frequency data increases. In other words, the evaluation value ANS increases when the final gaze point P is present in the specific region A, when the presence time of the gaze point P in the specific region A increases, when the arrival time at which the gaze point P arrives at the specific region A since the start time of the display period decreases, and when the number of times of movement of the gaze point P among the regions increases.

In contrast, the value of the evaluation value ANS decreases when the data value D1 of the final region data is set to 0, when the data value D2 of the presence time data decreases, when the data value D3 of the arrival time data increases, and when the data value D4 of the movement frequency data decreases. In other words, the evaluation value ANS decreases when the final gaze point P is not present in the specific region A, when the presence time of the gaze point P in the specific region A decreases, when the arrival time at which the gaze point P arrives at the specific region A since the start time of the display period increases, and when the number of times of movement of the gaze point P among the regions decreases.

Therefore, the evaluation unit 224 is able to obtain the evaluation data by determining whether the evaluation value ANS is equal to or larger than a predetermined value. For example, if the evaluation value ANS is equal to or larger than the predetermined value, it is possible to evaluate that the subject is less likely to have cognitive impairment and brain impairment. Further, if the evaluation value ANS is smaller than the predetermined value, it is possible to evaluate that the subject is highly likely to have cognitive impairment and brain impairment.

Furthermore, the evaluation unit 224 may obtain the evaluation value of the subject on the basis of at least one piece of data among the gaze point data as described above. For example, the evaluation unit 224 is able to evaluate that the subject is less likely to have cognitive impairment and brain impairment if the presence time data CNTA of the specific region A is equal to or larger than the predetermined value. Moreover, the evaluation unit 224 may perform evaluation by using the pieces of presence time data CNTB, CNTC, and CNTD of the comparison regions B to D. In this case, the evaluation unit 224 is able to evaluate that the subject is less likely to have cognitive impairment and brain impairment if a ratio of the presence time data CNTA of the specific region A and a sum of the pieces of presence time data CNTB, CNTC, and CNTD of the comparison regions B to D (a ratio of a gazing rate of the specific region A and gazing rates of the comparison regions B to D) is equal to or larger than a predetermined value. Furthermore, the evaluation unit 224 is able to evaluate that the subject is less likely to have cognitive impairment and brain impairment if a ratio of the presence time data CNTA of the specific region A and a total gazing time (a ratio of a gazing time of the specific region A and the total gazing time) is equal to or larger than a predetermined value. Moreover, the evaluation unit 224 is able to evaluate that the subject is less likely to have cognitive impairment and brain impairment if the final region is the specific region A, and that the subject is highly likely to have cognitive impairment and brain impairment if the final region is the comparison regions B to D.

Furthermore, the evaluation unit 224 is able to store the value of the evaluation value ANS in the storage unit 222. For example, it may be possible to cumulatively store the evaluation value ANS for the same subject, and perform evaluation by comparison with past evaluation values. For example, if the evaluation value ANS has a higher value than a past evaluation value, it is possible to evaluate that a cognitive function and a brain function have improved relative to those at the previous evaluation. Moreover, if a cumulative value of the evaluation value ANS is gradually increased for example, it is possible to evaluate that the cognitive function and the brain function have gradually improved.

Furthermore, the evaluation unit 224 may perform evaluation by using the presence time data, the movement frequency data, the final region data, and the arrival time data independently or in combination. For example, if the gaze point P accidentally arrives at the specific region A1 while a number of target objects are viewed, the data value D4 of the movement frequency data decreases. In this case, it is possible to perform evaluation by additionally using the data value D2 of the presence time data as described above. For example, even when the number of times of movement is small, if the presence time is long, it is possible to evaluate that the specific region A1 as the correct answer is gazed at. Moreover, if the number of times of movement is small and the presence time is short, it is possible to evaluate that the gaze point P has accidentally passed through the specific region A1.

Furthermore, when the number of times of movement is small, and if the final region is the specific region A1, it is possible to evaluate that the gaze point arrives at the specific region A1 that is the correct answer through a small number of times of movement, for example. In contrast, when the number of times of movement as described above is small, and if the final region is not the specific region A1, it is possible to evaluate that the gaze point P has accidentally passed through the specific region A1, for example.

In the present embodiment, when the evaluation unit 224 outputs the evaluation data, the output control unit 226 is able to cause the output device 50 to output character data indicating that "it seems that the subject is less likely to have cognitive impairment and brain impairment" or character data indicating that "it seems that the subject is highly likely to have cognitive impairment and brain impairment" in accordance with the evaluation data, for example. Further, if the evaluation value ANS has increased relative to a past evaluation value ANS of the same subject, the output control unit 226 is able to cause the output device 50 to output character data indicating that "a cognitive function and a brain function have improved" or the like.

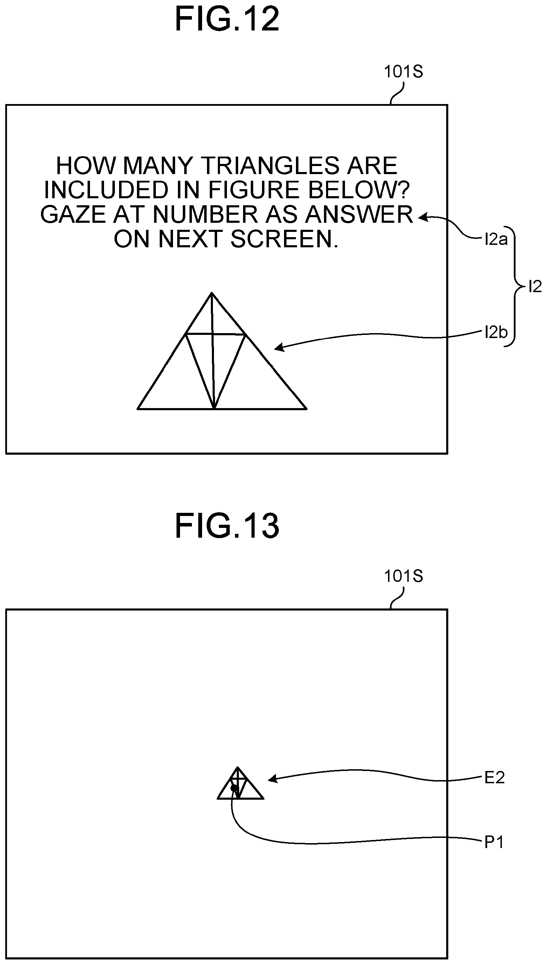

FIG. 12 is a diagram illustrating an example of the question information I2 displayed on the display screen 101S. As illustrated in FIG. 12, the display control unit 202 displays the question information I2 including character information I2a and figure information I2b on the display screen 101S in the first display operation. In the example illustrated in FIG. 12, the question information I2 is a question indicating an instruction to obtain the number of triangles included in a figure that is illustrated as the figure information I2b, and gaze at a correct number. In this case, the display control unit 202 displays, as the question information I2, both of the character information I2a and the figure information I2b.

FIG. 13 is a diagram illustrating an example of a guidance target object E2 displayed on the display screen 101S. As illustrated in FIG. 13, in the second display operation, the display control unit 202 displays a video of the guidance target object E2, which is obtained by reducing only the figure information I2b of the question information I2 toward the target position P1, as an eye-catching video on the display screen 101S. In this manner, the display control unit 202 is able to use only partial information of the question information I2 as the guidance target object E2.

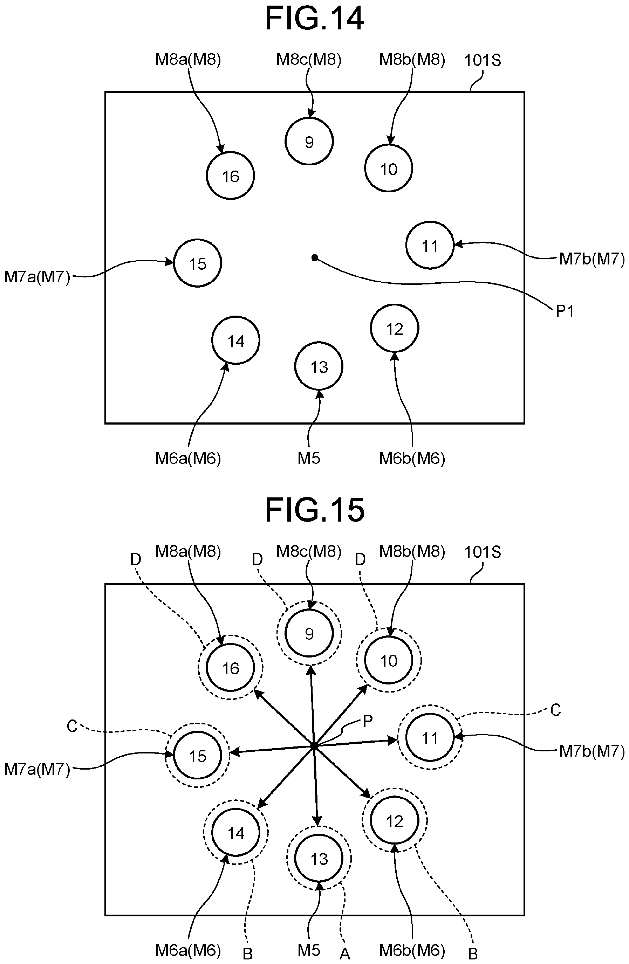

FIG. 14 is a diagram illustrating an example of answer target objects displayed on the display screen 101S. As illustrated in FIG. 14, as the third display operation, the display control unit 202 displays a plurality of answer target objects M5 to M8 that indicate respective numbers of "9" to "16" on the display screen 101S. The display control unit 202 displays, as the plurality of answer target objects M5 to M8, the specific target object M5 that is a correct answer for the question information I2 and the comparison target objects M6 to M8 that are different from the specific target object M5 and that are incorrect answers for the question information I2 on the display screen 101S.

Even in this case, the display control unit 202 arranges the plurality of answer target objects M5 to M8 at positions that do not overlap with one another. Further, the display control unit 202 arranges the plurality of answer target objects M5 to M8 at positions that do not overlap with the guidance position. For example, the display control unit 202 arranges the plurality of answer target objects M5 to M8 around the target position P1 that is the guidance position. The display control unit 202 may arrange the plurality of answer target objects M5 to M8 at radial positions at equal distances from the target position P1 that is the guidance position. For example, the display control unit 202 may arrange the plurality of answer target objects M5 to M8 at regular pitches on the same circumference of a circle centered at the target position P1.

FIG. 15 is a diagram illustrating an example of regions that are set on the display screen 101S during the display period in which the third display operation is performed. As illustrated in FIG. 15, the region setting unit 216 sets the specific region A corresponding to the specific target object M5 during the display period in which the third display operation is. Further, the region setting unit 216 sets the comparison regions B to D corresponding to the respective comparison target objects M6 to M8. In this case, the region setting unit 216 sets the specific region A and the comparison regions B to D at positions that do not overlap with one another.

The region setting unit 216 adopts the comparison region B for a comparison target object M6a indicating a number "14" and a comparison target object M6b indicating a number "12", for each of which a difference from a reference number "13" indicated by the specific target object M5 that is the correct answer is 1, for example. Further, the comparison region C is adopted for a comparison target object M7a indicating a number "15" and a comparison target object M7b indicating a number "11", for each of which the difference is 2. Furthermore, a comparison region D is adopted for a comparison target object M8a indicating a number "16", a comparison target object M8b indicating "10", and a comparison target object M8c indicating "9", for each of which the difference is 3 or more.

In this setting, when the data value D1 is obtained in evaluation, and if the final gaze point P of the subject is not present in the specific region A, it is possible to assign a certain data value in order of answer of the closest number to the correct answer, instead of setting the data value D1 to 0. For example, it may be possible to obtain 0.6 as the data value D1 if the final gaze point P of the subject is present in the comparison region B, 0.2 if the final gaze point P of the subject is present in the comparison region C, and 0 if the final gaze point P of the subject is present in the comparison region D.

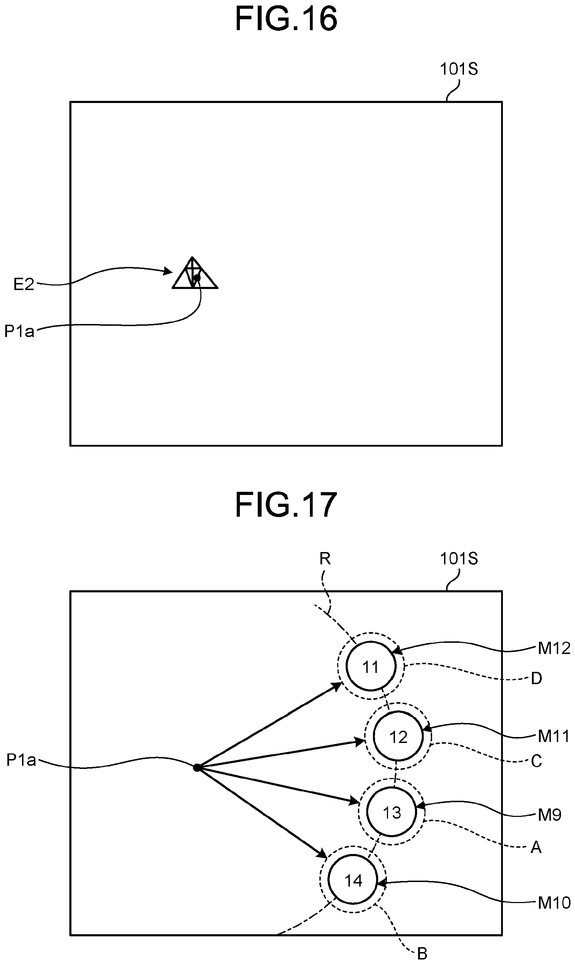

FIG. 16 is a diagram illustrating another display example of the guidance target object E2 displayed on the display screen 101S. As illustrated in FIG. 16, the display control unit 202 may set a target position P1a at a position deviated from the center of the display screen 101S. In this case, in the second display operation, the display control unit 202 displays a video of a guidance target object E2, which is obtained by reducing only the figure information I2b of the question information I2 toward the target position P1a, as an eye-catching video on the display screen 101S.

FIG. 17 is a diagram illustrating another display example of answer target objects displayed on the display screen 101S. As illustrated in FIG. 17, as the third display operation, the display control unit 202 displays a plurality of answer target objects M9 to M12 on the display screen 101S. The display control unit 202 displays, as the plurality of answer target objects M9 to M12, the specific target object M9 that is a correct answer for the question information I2 and the comparison target objects M10 to M12 that are different from the specific target object M9 and that are incorrect answers for the question information I2 on the display screen 101S.

Similarly to the above, the display control unit 202 arranges the plurality of answer target objects M9 to M12 at positions that do not overlap with one another. Further, the display control unit 202 arranges the plurality of answer target objects M9 to M12 at positions that do not overlap with the guidance position. For example, the display control unit 202 arranges the plurality of answer target objects M9 to M12 around the target position P1 that is the guidance position. The display control unit 202 may arrange the plurality of answer target objects M9 to M12 at positions at equal distances from the target position P1 that is the guidance position. For example, the display control unit 202 may arrange the plurality of answer target objects M9 to M12 at regular pitches on a circular arc R centered at the target position P1a. Further, as illustrated in FIG. 17, the region setting unit 216 sets the specific region A corresponding to the specific target object M9 during the display period in which the third display operation is performed. Furthermore, the region setting unit 216 sets the comparison regions B to D corresponding to the respective comparison target objects M10 to M12. In this case, the region setting unit 216 sets the specific region A and the comparison regions B to D at positions that do not overlap with one another.

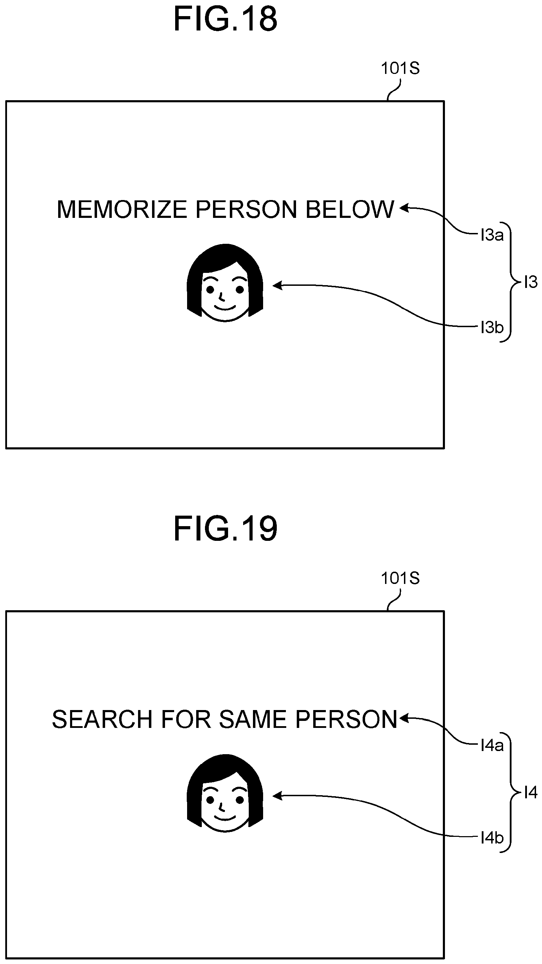

FIG. 18 is a diagram illustrating an example of the instruction information displayed on the display screen 101S. As illustrated in FIG. 18, before performing the first display operation, the display control unit 202 is able to display instruction information I3 for instructing the subject to memorize information that is to be a premise of question information. The instruction information I3 includes image information I3b indicating a face of a person and character information I3a indicating an instruction to memorize the face of the person indicated by the image information I3b.

FIG. 19 is a diagram illustrating an example of the question information displayed on the display screen 101S. As illustrated in FIG. 19, after displaying the instruction information I3 for a certain time, the display control unit 202 displays question information I4 for the subject as the first display operation. In the example illustrated in FIG. 19, the question information I4 is a question indicating an instruction to gaze at the same person as the face of the person indicated by the image information I3b. The question information I4 includes character information I4a indicating contents of the above-described question and image information I4b indicating the same image as the image information I3b.

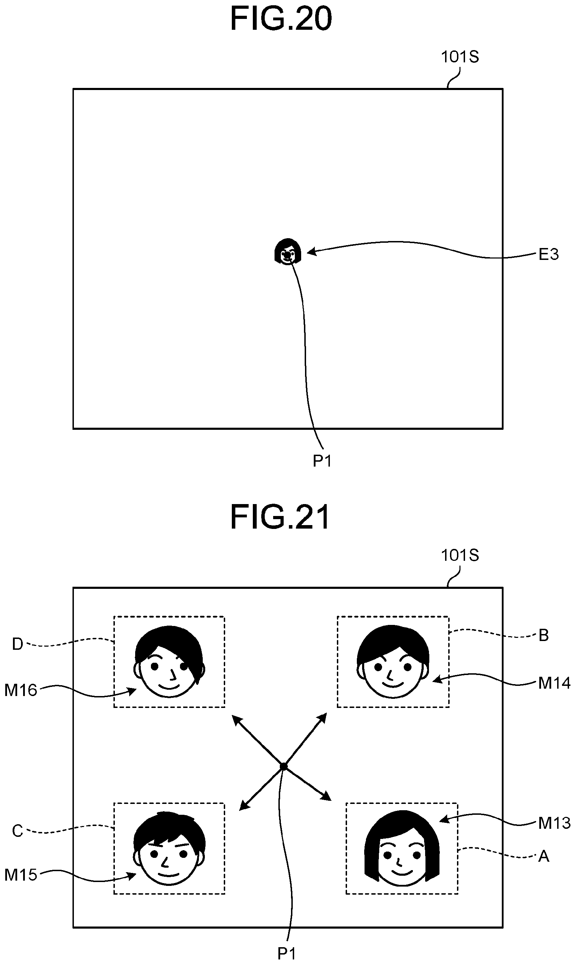

FIG. 20 is a diagram illustrating an example of a guidance target object E3 displayed on the display screen 101S. As illustrated in FIG. 20, in the second display operation, the display control unit 202 displays a video of the guidance target object E3, which is obtained by reducing only the image information I4b of the question information I4 toward the target position P1, as an eye-catching video on the display screen 101S. In this manner, the display control unit 202 is able to use partial information of the question information I4 as the guidance target object E3.

FIG. 21 is a diagram illustrating an example of answer target objects displayed on the display screen 101S. As illustrated in FIG. 21, as the third display operation, the display control unit 202 displays a plurality of answer target objects M13 to M16 indicating images of faces of different persons on the display screen 101S. The display control unit 202 displays, as the plurality of answer target objects M13 to M16, the specific target object M13 that is a correct answer for the question information I4 and the comparison target objects M14 to M16 that are different from the specific target object M13 and that are incorrect answers for the question information I4 on the display screen 101S. Meanwhile, the image of the specific target object M13 that is the correct answer is the same as the images of the image information I3b and the image information I4b as described above. The display control unit 202 arranges the plurality of answer target objects M13 to M16 at positions that do not overlap with one another. Further, the display control unit 202 arranges the plurality of answer target objects M13 to M16 at positions that do not overlap with the guidance position. For example, the display control unit 202 arranges the plurality of answer target objects M13 to M16 around the guidance position. In the present embodiment, the guidance position is the target position P1 to which the gaze point of the subject is guided by the guidance target object E3. The display control unit 202 may arrange the plurality of answer target objects M13 to M16 at positions at equal distances from the target position P1 that is the guidance position.

Furthermore, FIG. 21 also illustrates an example of regions that are set on the display screen 101S during the display period in which the third display operation is performed. As illustrated in FIG. 21, the region setting unit 216 sets the specific region A corresponding to the specific target object M13 during the display period in which the third display operation is performed. Moreover, the region setting unit 216 sets the comparison regions B to D corresponding to the respective comparison target objects M14 to M16. In this case, the region setting unit 216 sets the specific region A and the comparison regions B to D at positions that do not overlap with one another.

It is known that symptoms of cognitive impairment and brain impairment affect cognitive ability. If the subject does not have cognitive impairment and brain impairment, the subject is able to view, one by one, the comparison target objects M14 to M16 that are displayed on the display screen 101S in the third display operation, determine, by comparison, that the comparison target objects M14 to M16 are not the same as the person indicated by the image information I4b that is memorized in the first display operation, and finally detect and gaze at the specific target object M13. In contrast, if the subject has cognitive impairment and brain impairment, in some cases, it may be difficult to memorize the specific target object M13 or immediately forget the specific target object M13 after memorizing it. Therefore, in some cases, it may be difficult to perform comparison as described above, and it may be difficult to gaze at the specific target object M13. In the present embodiment, it is possible to prevent the gaze point of the subject from moving to or being fixed to any of the answer target objects M13 to M16 at the start of the third display operation, so that it is possible to evaluate memory ability of the subject with high accuracy.

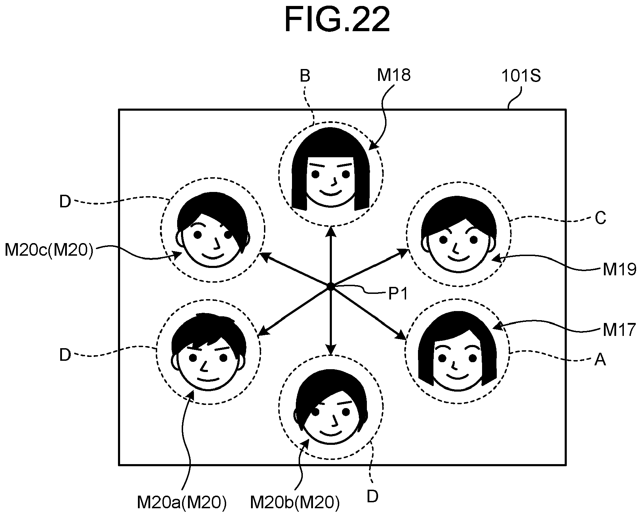

FIG. 22 is a diagram illustrating another display example of answer target objects displayed on the display screen 101S. As illustrated in FIG. 22, the display control unit 202 may arrange a plurality of answer target objects M17 to M20 at radial positions at equal distances from the target position P1 that is the guidance position. For example, the display control unit 202 is able to arrange the plurality of answer target objects M17 to M20 at regular pitches on the same circumference of a circle centered at the target position P1. Furthermore, FIG. 22 also illustrates an example of regions that are set on the display screen 101S during the display period in which the third display operation is performed. As illustrated in FIG. 22, the region setting unit 216 sets the specific region A corresponding to the specific target object M17. Moreover, the region setting unit 216 adopts a certain characteristic (gender, facial expression, or the like) of the specific target object M17 that is a correct answer as a reference, and adopts, as the comparison region B, the comparison target object M18 for which the same gender, i.e., female, as the specific target object M17 is set. Furthermore, the comparison target object M19, for which male is set as gender but which has a relatively large number of common appearances, such as eyebrows and a nose shape, in facial expression, is adopted as the comparison region C. Moreover, the comparison target objects M20 (M20a to M20c), for each of which male is set as gender and which has a small number of common appearances in facial expression, is adopted as the comparison region D. In this case, the region setting unit 216 sets the specific region A and the comparison regions B to D at positions that do not overlap with one another.

In this setting, when the data value D1 is obtained in evaluation, and if the final gaze point P of the subject is not present in the specific region A, it is possible to assign a certain data value in order of answer of the closest number to the correct answer, instead of setting the data value D1 to 0. For example, it may be possible to obtain 0.6 as the data value D1 if the final gaze point P of the subject is present in the comparison region B, 0.2 if the final gaze point P of the subject is present in the comparison region C, and 0 if the final gaze point P of the subject is present in the comparison region D.

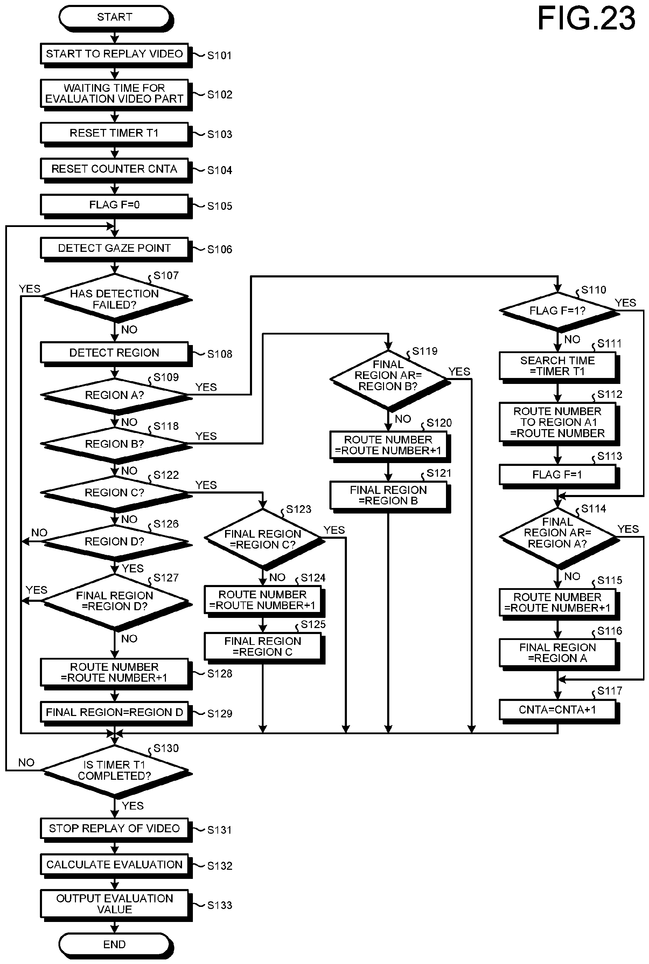

Next, an example of the evaluation method according to the present embodiment will be described with reference to FIG. 23. FIG. 23 is a flowchart illustrating an example of the evaluation method according to the present embodiment. In the present embodiment, the display control unit 202 starts to replay a video (Step S101). After a lapse of a waiting time for an evaluation video part (Step S102), the timer T is reset (Step S103), the count value CNTA of the counter is reset (Step S104), and the flag value is set to 0 (Step S105).

The gaze point detection unit 214 detects the positional data of the gaze point of the subject on the display screen 101S of the display device 101 with a defined sampling period (for example, 20 msec) while showing the video displayed on the display device 101 to the subject (Step S106). If the positional data is detected (No at Step S107), the determination unit 218 determines a region in which the gaze point P is present on the basis of the positional data (Step S108). Further, if the positional data is not detected (Yes at Step S107), processes from Step S130 to be described later are performed.

If it is determined that the gaze point P is present in the specific region A (Yes at Step S109), the arithmetic unit 220 determines whether the flag value is set to 1, that is, whether the gaze point P arrived at the task region A for the first time (1: has already arrived, 0: has not arrived yet) (Step S110). If the flag value is set to 1 (Yes at Step S110), the arithmetic unit 220 skips Step S111 to Step S113 to be described below, and performs a process at Step S114 to be described later.

Further, if the flag value is not set to 1, that is, if the gaze point P arrived at the specific region A for the first time (No at Step S110), the arithmetic unit 220 extracts a measurement result of the timer T as the arrival time data (Step S111). Furthermore, the arithmetic unit 220 stores movement frequency data, which indicates the number of times of movement of the gaze point P among the regions before the gaze point P arrives at the specific region A, in the storage unit 222 (Step S112). Thereafter, the arithmetic unit 220 changes the flag value to 1 (Step S113) .

Subsequently, the arithmetic unit 220 determines whether a region in which the gaze point P is present at the last detection, that is, the final region, is the specific region A (Step S114). If it is determined that the final region is the specific region A1 (Yes at Step S114), the arithmetic unit 220 skips Step S115 and Step S116 to be described below, and performs a process at Step S117 to be described later. Furthermore, if it is determined that the final region is not the specific region A1 (No at Step S114), the arithmetic unit 220 increments the cumulative number, which indicates the number of times of movement of the gaze point P among the regions, by 1 (Step S115), and changes the final region to the specific region A (Step S116). Moreover, the arithmetic unit 220 increments the count value CNTA, which indicates the presence time data in the specific region A, by 1 (Step S117). Thereafter, the arithmetic unit 220 performs the processes from Step S130 to be described later.

Furthermore, if it is determined that the gaze point P is not present in the specific region A (No at Step S109), the arithmetic unit 220 determines whether the gaze point P is present in the comparison region B (Step S118). If it is determined that the gaze point P is present in the comparison region B (Yes at Step S118), the arithmetic unit 220 determines whether the region in which the gaze point P is present at the last detection, that is, the final region, is the comparison region B (Step S119). If it is determined that the final region is the comparison region B (Yes at Step S119), the arithmetic unit 220 skips Step S120 and Step S121 to be described below, and performs the process at Step S130 to be described later. Moreover, if it is determined that the final region is not the comparison region B (No at Step S119), the arithmetic unit 220 increments the cumulative number, which indicates the number of times of movement of the gaze point P among the regions, by 1 (Step S120), and changes the final region to the comparison region B (Step S121). Thereafter, the arithmetic unit 220 performs the processes from Step S130 to be described later.

Furthermore, if it is determined that the gaze point P is not present in the comparison region B (No at Step S118), the arithmetic unit 220 determines whether the gaze point P is present in the comparison region C (Step S122). If it is determined that the gaze point P is present in the comparison region C (Yes at Step S122), the arithmetic unit 220 determines whether the region in which the gaze point P is present at the last detection, that is, the final region, is the comparison region C (Step S123). If it is determined that the final region is the comparison region C (Yes at Step S123), the arithmetic unit 220 skips Step S124 and Step S125 to be described below, and performs the process at Step S130 to be described later. Moreover, if it is determined that the final region is not the comparison region C (No at Step S123), the arithmetic unit 220 increments the cumulative number, which indicates the number of times of movement of the gaze point P among the regions, by 1 (Step S124), and changes the final region to the comparison region C (Step S125). Thereafter, the arithmetic unit 220 performs the processes from Step S130 to be described later.

Furthermore, if it is determined that the gaze point P is not present in the comparison region C (No at Step S122), the arithmetic unit 220 determines whether the gaze point P is present in the comparison region D (Step S126). If it is determined that the gaze point P is present in the comparison region D (Yes at Step S126), the arithmetic unit 220 determines whether the region in which the gaze point P is present at the last detection, that is, the final region, is the comparison region D (Step S127). Moreover, if it is determined that the gaze point P is not present in the comparison region D (No at Step S126), the process at Step S130 to be described later is performed. Furthermore, if it is determined that the final region is the comparison region D (Yes at Step S127), the arithmetic unit 220 skips Step S128 and Step S129 to be described below, and performs the process at Step S130 to be described later. Moreover, if it is determined that the final region is not the comparison region D (No at Step S127), the arithmetic unit 220 increments the cumulative number, which indicates the number of times of movement of the gaze point P among the regions, by 1 (Step S128), and changes the final region to the comparison region D (Step S129). Thereafter, the arithmetic unit 220 performs the processes from Step S130 to be described later.

Thereafter, the arithmetic unit 220 determines whether a time at which replay of the video is completed has come, on the basis of a detection result of the detection timer T (Step S130). If the arithmetic unit 220 determines that the time at which replay of the video is completed has not yet come (No at Step S130), the arithmetic unit 220 repeats the processes from Step S106 as described above.

If the arithmetic unit 220 determines that the time at which replay of the video is completed has come (Yes at Step S130), the display control unit 202 stops replay of the video (Step S131). After replay of the video is stopped, the evaluation unit 224 calculates the evaluation value ANS on the basis of the presence time data, the movement frequency data, the final region data, and the arrival time data that are obtained from processing results as described above (Step S132), and obtains evaluation data on the basis of the evaluation value ANS. Thereafter, the output control unit 226 outputs the evaluation data obtained by the evaluation unit 224 (Step S133).

As described above, the evaluation apparatus according to the present embodiment includes the display screen 101S, the gaze point detection unit that detects a position of a gaze point of a subject who observes an image displayed on the display screen 101S, the display control unit 202 that performs display operation including the first display operation of displaying question information that is a question for the subject on the display screen 101S, the second display operation of displaying a guidance target object that guides the gaze point P of the subject to the predetermined target position P1 on the display screen 101S, and the third display operation of displaying a plurality of answer target objects, which are answers for the question, at positions that do not overlap with the guidance position on the display screen 101S after the second display operation, the region setting unit 216 that sets the specific region A corresponding to the specific target object among the plurality of answer target objects and the comparison regions B to D corresponding to the comparison target objects that are different from the specific target object, the determination unit 218 that determines whether the gaze point P is present in the specific region A and the comparison regions B to D during the display period in which the third display operation is performed, on the basis of the position of the gaze point P, the arithmetic unit 220 that calculates gaze point data during the display period on the basis of a determination result, and the evaluation unit 224 that obtains evaluation data of the subject on the basis of the gaze point data.

Furthermore, the evaluation method according to the present embodiment includes detecting a position of a gaze point of a subject who observes an image displayed on the display screen 101S, performing display operation including the first display operation of displaying question information that is a question for the subject on the display screen 101S, the second display operation of displaying a guidance target object that guides the gaze point P of the subject to the predetermined target position P1 on the display screen 101S, and the third display operation of displaying a plurality of answer target objects, which are answers for the question, at positions that do not overlap with the guidance position on the display screen 101S after the second display operation, setting the specific region A corresponding to the specific target object among the plurality of answer target objects and the comparison regions B to D corresponding to the comparison target objects that are different from the specific target object, determining whether the gaze point P is present in the specific region A and the comparison regions B to D during the display period in which the third display operation is performed, on the basis of the position of the gaze point P, calculating gaze point data during the display period on the basis of a determination result, and obtaining evaluation data of the subject on the basis of the gaze point data.

Moreover, the evaluation program according to the present embodiment causes a computer to execute a process of detecting a position of a gaze point of a subject who observes an image displayed on the display screen 101S, a process of performing display operation including the first display operation of displaying question information that is a question for the subject on the display screen 101S, the second display operation of displaying a guidance target object that guides the gaze point P of the subject to the predetermined target position P1 on the display screen 101S, and the third display operation of displaying a plurality of answer target objects, which are answers for the question, at positions that do not overlap with the guidance position on the display screen 101S after the second display operation, a process of setting the specific region A corresponding to the specific target object among the plurality of answer target objects and the comparison regions B to D corresponding to the comparison target objects that are different from the specific target object, a process of determining whether the gaze point P is present in the specific region A and the comparison regions B to D during the display period in which the third display operation is performed, on the basis of the position of the gaze point P, a process of calculating gaze point data during the display period on the basis of a determination result, and a process of obtaining evaluation data of the subject on the basis of the gaze point data.

According to the present embodiment, it is possible to prevent the gaze point of the subject from moving to or being fixed to any of the answer target objects at the start of the third display operation, so that it is possible to reduce contingency and evaluate the subject with high accuracy. Furthermore, it is possible to obtain the evaluation data of the subject on the basis of the course of movement of the gaze point during the display period, so that it is possible to evaluate the subject with higher accuracy. Therefore, the evaluation apparatus 100 is able to evaluate the subject with high accuracy.

Furthermore, in the evaluation apparatus 100 according to the present embodiment, the region setting unit 216 sets the specific region A and the comparison regions B to D at positions that do not overlap with one another. Therefore, it is possible to distinguish an answer of the subject with high accuracy, so that it is possible to evaluate the subject with high accuracy.

Moreover, in the evaluation apparatus 100 according to the present embodiment, the display control unit 202 arranges the plurality of answer target objects at positions at equal distances from the guidance position. Therefore, it is possible to further reduce contingency and distinguish an answer of the subject with high accuracy.

Furthermore, in the evaluation apparatus 100 according to the present embodiment, the movement course data includes at least one of the arrival time data that indicates a time period from the start time of the display period to an arrival time at which the gaze point first arrives at the specific region A, the movement frequency data that indicates the number of times of movement of the position of the gaze point P among the plurality of comparison regions B to D before the gaze point P first arrives at the specific region A, and the presence time data that indicates a presence time in which the gaze point P is present in the specific region A during the display period, and also includes the final region data that indicates a region in which the gaze point P is present among the specific region A and the comparison regions B to D during the display time. Therefore, it is possible to effectively obtain the evaluation data with high accuracy.

Moreover, in the evaluation apparatus 100 according to the present embodiment, the evaluation unit 224 adds weight to at least a single piece of data included in the movement course data and obtains the evaluation data. Therefore, by giving priority to each piece of data, it is possible to obtain the evaluation data with high accuracy.

The technical scope of the present invention is not limited to the embodiment as described above, and it is possible to apply modifications appropriately within a scope not departing from the gist of the present invention.

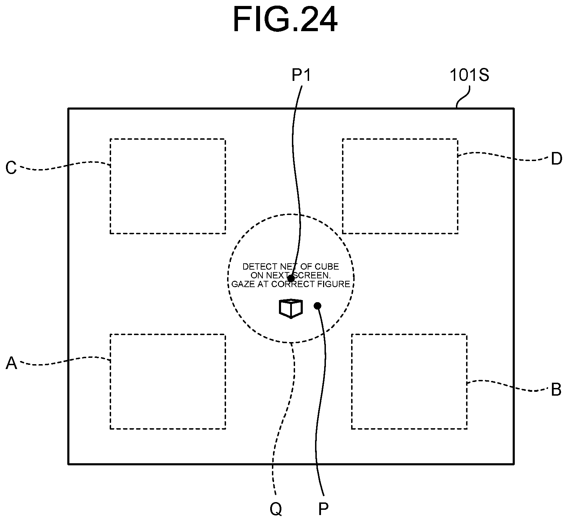

FIG. 24 is a diagram illustrating an example of operation that is performed after the second display operation is performed. As illustrated in FIG. 24, the determination unit 218 may detect whether the gaze point is present in a predetermined region Q including the target position P1 after the second display operation, on the basis of the positional data of the gaze point, and perform determination if it is detected that the gaze point is present in the predetermined region Q. The predetermined region Q may be set to a range that includes the target position P1 and that does not overlap with the specific region A and the comparison regions B to D that are set in the third display operation, for example.

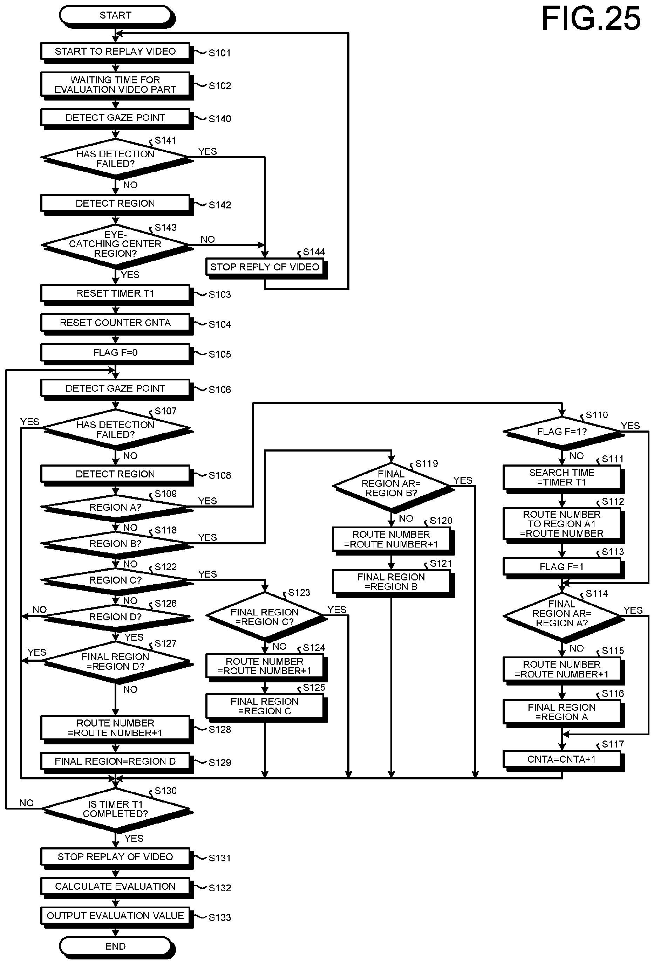

FIG. 25 is a flowchart illustrating another example of the evaluation method according to the present embodiment. FIG. 25 illustrates operation of performing determination when it is detected that the gaze point is present in the predetermined region Q. As illustrated in FIG. 25, similarly to the embodiment as described above, the display control unit 202 starts to replay the video (Step S101). After a lapse of a waiting time for an evaluation video part (Step S102), the gaze point detection unit 214 detects the positional data of the gaze point of the subject on the display screen 101S of the display device 101 with a defined sampling period (for example, 20 msec) while showing the video displayed on the display device 101 to the subject (Step S140). If the positional data is detected (No at Step S141), the determination unit 218 detects a region in which the gaze point P is present on the basis of the positional data (Step S142).

If it is determined that the gaze point P is present in the predetermined region Q (Yes at Step S143), the timer T is reset (Step S103), the count value CNTA of the counter is reset (Step S104), and the flag value is set to 0 (Step S105). Then, the processes from Step S106 are performed. Furthermore, if the positional data is not detected (Yes at Step S141), and if it is determined that the gaze point P is not present in the predetermined region Q (No at Step S143), replay of the video is stopped (Step S144), and the processes from Step S101 are repeated. Therefore, it is possible to more reliably locate the gaze point P of the subject at the target position P1 or the predetermined region Q around the target position P1.

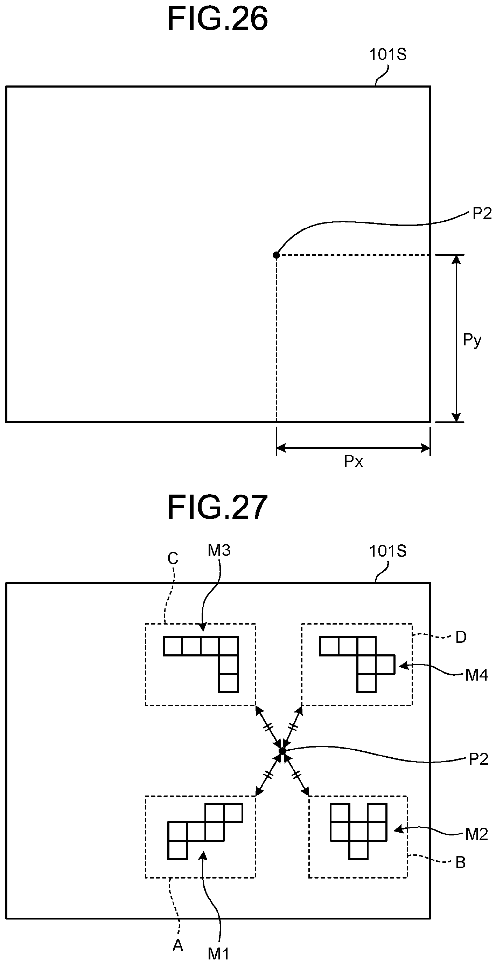

Moreover, FIG. 26 is a diagram illustrating another example of operation that is performed after the second display operation is performed. As illustrated in FIG. 26, the gaze point detection unit 214 may obtain a position of the gaze point P after the second display operation, that is, the position of the gaze point P that is guided by the guidance target object, on the basis of the positional data of the gaze point. The position of the gaze point P can be obtained based on an X coordinate (Px) and a Y coordinate (Py) with reference to the position of an origin of the display screen 101S (for example, a lower right corner portion in the figure), for example. The gaze point detection unit 214 sets the obtained position of the gaze point P as a calculated position P2.

FIG. 27 is a diagram illustrating an example of answer target objects displayed on the display screen 101S. As illustrated in FIG. 27, as the third display operation, the display control unit 202 arranges the plurality of answer target objects M1 to M4 around the guidance position. In this case, the calculated position P2 that is calculated after the second display operation is adopted as the guidance position. The display control unit 202 may arrange the plurality of answer target objects M1 to M4 at positions at equal distances from the calculated position P2 that is the guidance position. Furthermore, the region setting unit 216 sets the specific region A corresponding to the specific target object M1 arranged as described above, and sets the comparison regions B to D corresponding to the respective comparison target objects M2 to M4.

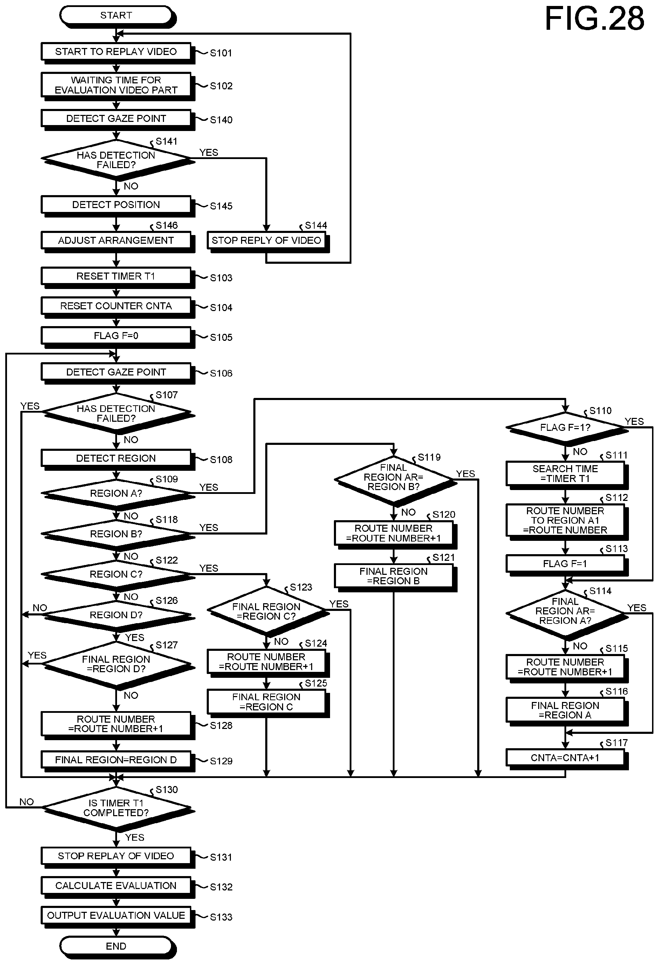

FIG. 28 is a flowchart illustrating another example of the evaluation method according to the present embodiment. FIG. 28 illustrates operation of calculating the position of the gaze point P and arranging the answer target objects M1 to M4 while adopting the calculated position P2 as the guidance position. As illustrated in FIG. 28, similarly to the embodiment as described above, the display control unit 202 starts to replay the video (Step S101). After a lapse of a waiting time for an evaluation video part (Step S102), the gaze point detection unit 214 detects the positional data of the gaze point of the subject on the display screen 101S of the display device 101 with a defined sampling period (for example, 20 msec) while showing the video displayed on the display device 101 to the subject (Step S140). If the positional data is detected (No at Step S141), the determination unit 218 sets the calculated position P2 that is the position of the gaze point P, on the basis of the positional data (Step S145). If the determination unit 218 sets the calculated position P2, the display control unit 202 arranges the plurality of answer target objects M1 to M4 around the calculated position P2 (Step S146). If the plurality of answer target objects M1 to M4 are arranged, the timer T is reset (Step S103), the count value CNTA of the counter is reset (Step S104), and the flag value is set to 0 (Step S105). Then, the processes from Step S106 are performed. Furthermore, if the positional data is not detected (Yes at Step S141), replay of the video is stopped (Step S144), and the processes from Step S101 are repeated. Therefore, the positions of the plurality of answer target objects M1 to M4 are set based on the position of the gaze point P of the subject P after the second display operation, so that it is possible to more reliably prevent the gaze point P of the subject from being arranged on the plurality of answer target objects M1 to M4 at the start of the third display operation.

Moreover, in the embodiments as described above, a case has been described as one example in which the evaluation apparatus 100 is used as an evaluation apparatus that evaluates the possibility of cognitive impairment and brain impairment, but embodiments are not limited to this example. For example, the evaluation apparatus 100 may be used as an evaluation apparatus that evaluates a subject who has development disability, rather than cognitive impairment and brain impairment.

Furthermore, the question information that is displayed on the display screen 101S in the first display operation is not limited to the question information indicating an instruction to gaze at a correct figure or a correct number for the question in the embodiments as described above. The question information may be a question that, for example, instructs the subject to memorize a number of figures that match a predetermined condition among a plurality of figures, and instructs the subject to perform a calculation using the memorized number.

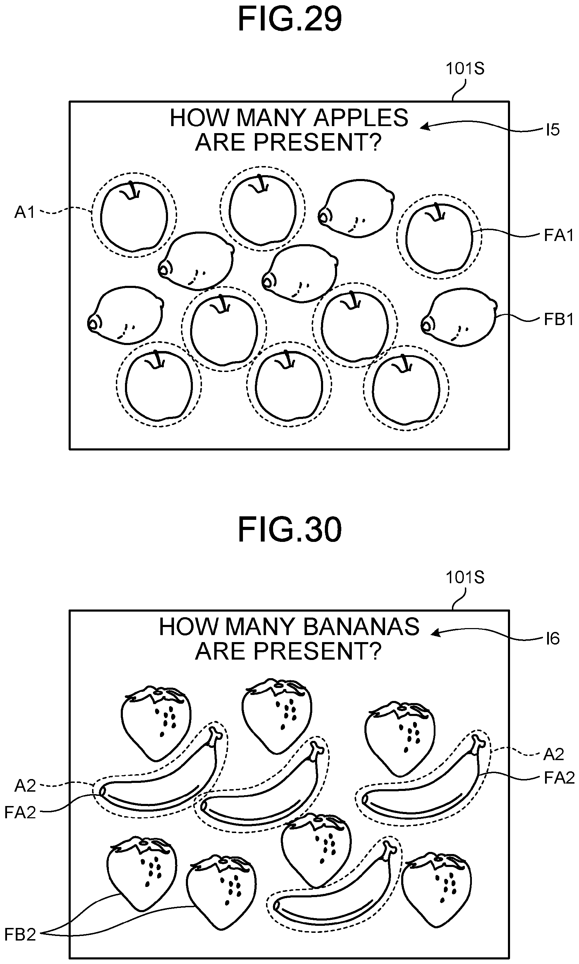

FIG. 29 to FIG. 31 are diagrams illustrating examples of question information displayed on the display screen 101S. As illustrated in FIG. 29, the display control unit 202 displays question information I5, a plurality of apple graphic images FA1, and a plurality of lemon graphic images FB1 on the display screen 101S. The question information I5 is a question indicating an instruction to obtain the number of the apple graphic images FA1 among the plurality of images and to memorize the number. Further, the region setting unit 216 sets a corresponding region A1 that corresponds to the apple graphic images FA1. The region setting unit 216 may set the corresponding region A1 in a region including at least a part of the apple graphic images FA1.

The display control unit 202 displays the question information I5, the plurality of apple graphic images FA1, and the plurality of lemon graphic images FB1 as described above on the display screen 101S for a predetermined period. During this period, the gaze point detection unit 214 detects the positional data of the gaze point of the subject on the display screen 101S of the display device 101 with a defined sampling period (for example, 20 msec). If the positional data of the gaze point P is detected, the determination unit 218 determines whether the gaze point of the subject is present in the corresponding region A1 with the above-described sampling period, and outputs determination data. The arithmetic unit 220 calculates first gaze time data indicating a gaze time for the apple graphic images FA1 indicated by the question information I5, on the basis of the determination data.

After displaying the question information I5, the plurality of apple graphic images FA1, and the plurality of lemon graphic images FB1 on the display screen 101S for the predetermined period, the display control unit 202 displays question information 16, a plurality of banana graphic images FA2, and a plurality of strawberry graphic images FB2 on the display screen 101S as illustrated in FIG. 30. The question information I6 is a question indicating an instruction to obtain the number of the banana graphic images FA2 among the plurality of images and to memorize the number. Further, the region setting unit 216 sets a corresponding region A2 that corresponds to the banana graphic images FA2. The region setting unit 216 may set the corresponding region A2 in a region including at least a part of the banana graphic images FA2.

The display control unit 202 displays the question information 16, the plurality of banana graphic images FA2, and the plurality of strawberry graphic images FB2 as described above on the display screen 101S for a predetermined period. During this period, the gaze point detection unit 214 detects the positional data of the gaze point of the subject on the display screen 101S of the display device 101 with a defined sampling period (for example, 20 msec). If the positional data of the gaze point P is detected, the determination unit 218 determines whether the gaze point of the subject is present in the corresponding region A2 with the above-described sampling period, and outputs determination data. The arithmetic unit 220 calculates second gaze time data indicating a gaze time for the banana graphic images FA2 indicated by the question information 16, on the basis of the determination data.

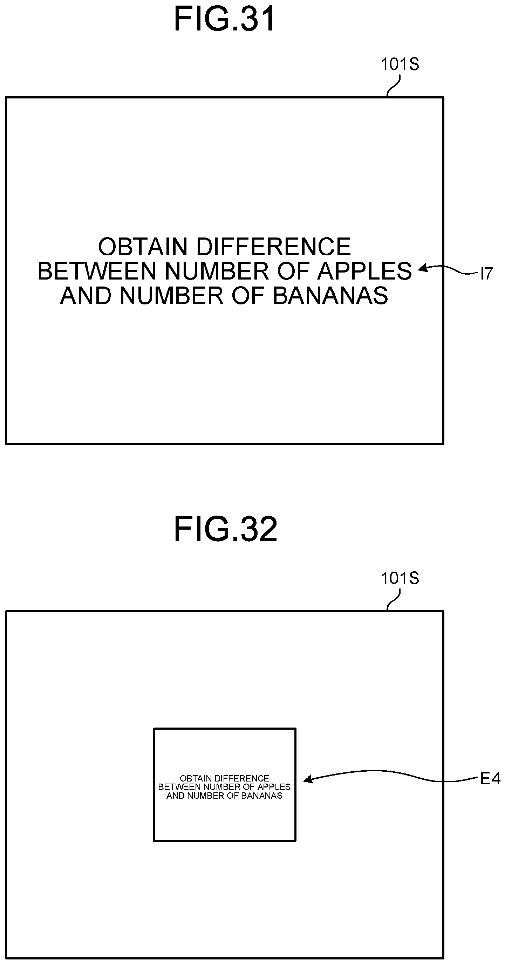

After displaying the question information 16, the plurality of banana graphic images FA2, and the plurality of strawberry graphic images FB2 on the display screen 101S for the predetermined period, the display control unit 202 displays, as question information 17, a question for instructing the subject to calculate a difference between the number of apples and the number of bananas on the display screen 101S as illustrated in FIG. 31.

After displaying the question information I7 on the display screen 101S for a predetermined period, the display control unit 202 displays, as the second display operation, a guidance target object on the display screen 101S. FIG. 32 is a diagram illustrating another example of a guidance target object displayed on the display screen 101S. As illustrated in FIG. 32, in the second display operation, the display control unit 202 displays a video of a guidance target object E4, which is obtained by reducing an entire image including the above-described question information I7 toward a predetermined target position on the display screen 101S, as an eye-catching video on the display screen 101S. In this example, the target position is set at the position of the center of the display screen 101S, but embodiments are not limited to this example.

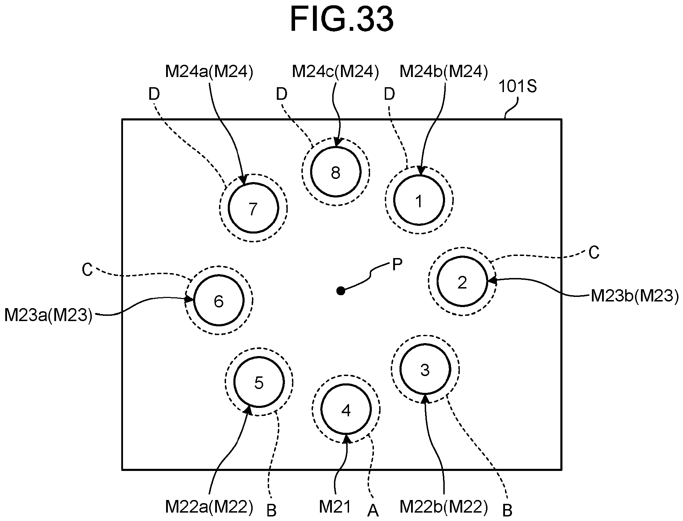

After performing the second display operation, the display control unit 202 performs the third display operation. FIG. 33 is a diagram illustrating another example of answer target objects displayed on the display screen 101S. As illustrated in FIG. 33, as the third display operation, the display control unit 202 displays a plurality of answer target objects M21 to M24 that indicate respective numbers of "1" to "8" on the display screen 101S. The display control unit 202 displays, as the plurality of answer target objects M21 to M24, the specific target object M21 that is a correct answer for the question information I7 and the comparison target objects M22 to M24 that are different from the specific target object M21 and that are incorrect answers for the question information I7 on the display screen 101S.

The display control unit 202 arranges the plurality of answer target objects M21 to M24 at positions that do not overlap with one another. Furthermore, the display control unit 202 arranges the plurality of answer target objects M21 to M24 at positions that do not overlap with the guidance position. For example, the display control unit 202 arranges the plurality of answer target objects M21 to M24 around the target position that is the guidance position. The display control unit 202 may arrange the plurality of answer target objects M21 to M24 at radial positions at equal distances from the target position that is the guidance position. For example, the display control unit 202 may arrange the plurality of answer target objects M21 to M24 at regular pitches on the same circumference of a circle centered at the target position.

During the display period in which the third display operation is performed, the region setting unit 216 sets the specific region A corresponding to the specific target object M21. Further, the region setting unit 216 sets the comparison regions B to D corresponding to the respective comparison target objects M22 to M24. In this case, the region setting unit 216 sets the specific region A and the comparison regions B to D at positions that do not overlap with one another.

The region setting unit 216 adopts the comparison region B for a comparison target object M22a indicating a number "5" and a comparison target object M22b indicating a number "3", for each of which a difference from a reference number "4" indicated by the specific target object M21 that is the correct answer is 1, for example. Further, the comparison region C is adopted for a comparison target object M23 indicating a number "6" and a comparison target object M23b indicating a number "2", for each of which the difference is 2. Furthermore, the comparison region D is adopted for the comparison target object M24a indicating a number "1" and a comparison target object M24c indicating a number "8", for each of which the difference is 3 or more.

In this setting, similarly to the embodiment as described above, when the data value D1 is obtained in evaluation, and if the final gaze point P of the subject is not present in the specific region A, it is possible to assign a certain data value in order of answer of the closest number to the correct answer, instead of setting the data value D1 to 0. For example, it may be possible to obtain 0.6 as the data value D1 if the final gaze point P of the subject is present in the comparison region B, 0.2 if the final gaze point P of the subject is present in the comparison region C, and 0 if the final gaze point P of the subject is present in the comparison region D.

In the third display operation, the gaze point detection unit 214 detects the positional data of the gaze point of the subject on the display screen 101S of the display device 101 with a defined sampling period (for example, 20 msec). If the positional data of the gaze point P is detected, the determination unit 218 determines whether the gaze point of the subject is present in the specific region A and the plurality of comparison regions B to D with the above-described sampling period, and outputs determination data. The arithmetic unit 220 calculates the movement course data that indicates the course of movement of the gaze point P during the display period, on the basis of the determination data. The arithmetic unit 220 calculates, as the movement course data, the presence time data, the movement frequency data, the final region data, and the arrival time data.

The evaluation unit 224 obtains the evaluation data by using the first gaze time data and the second gaze time data that are obtained in the first display operation and using the presence time data, the movement frequency data, the final region data, and the arrival time data that are obtained in the third display operation. Meanwhile, the evaluation data is obtained based on the presence time data, the movement frequency data, the final region data, and the arrival time data that are obtained in the third display operation, similarly to the embodiment as described above.

A subject who is highly likely to have cognitive impairment and brain impairment tends not to carefully view the figures indicated by the question information I5 and the question information I6. Further, a subject who is less likely to have cognitive impairment and brain impairment tends to carefully view the figures indicated by the question information I5 and the question information I6 in accordance with the question displayed on the display screen 101S. Accordingly, by referring to the first gaze time data and the second gaze time data that are obtained in the first display operation, it is possible to reflect the the gaze time for the figures indicated by the question information in the evaluation.

Therefore, assuming that a sum of the first gaze time data and the second gaze time data is denoted by D5, the evaluation value ANS is represented as follows, for example.

$$ANS = D1 \times K1 + D2 \times K2 + D3 \times K3 + D4 \times K4 + D5 \times K5$$

Meanwhile, D1 to D4 and K1 to K4 are the same as those of the embodiment as described above. Further, K5 is a constant for weighting, and can be set appropriately similarly to K1 to K4. It may be possible to appropriately set an upper limit value for the data value D5. Further, the evaluation unit 224 may obtain the evaluation value of the subject on the basis of at least a single piece of data in the gaze point data, similarly to the embodiment as described above.

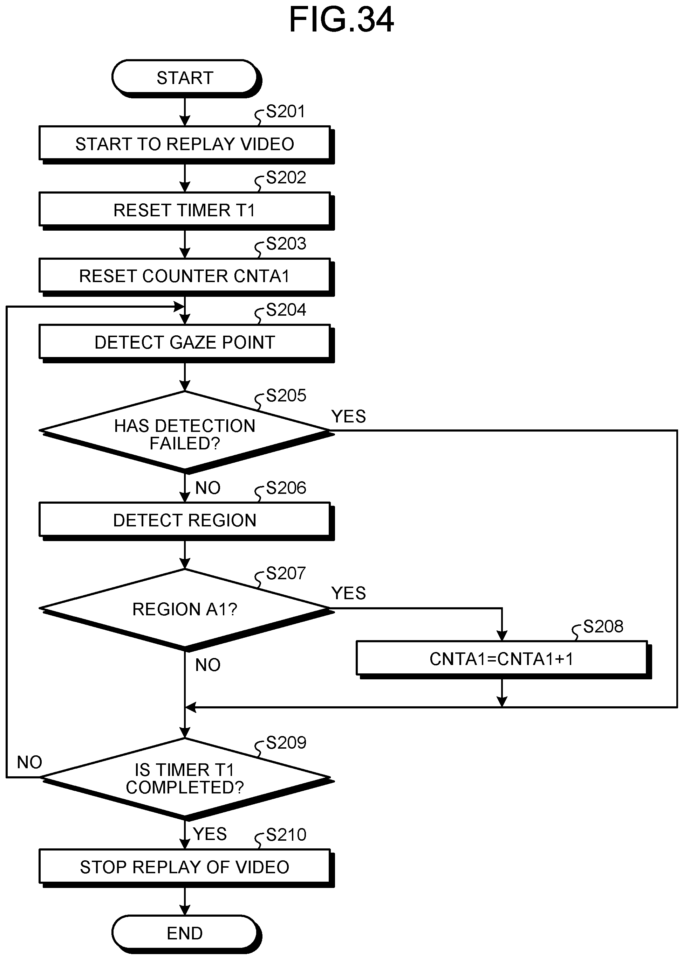

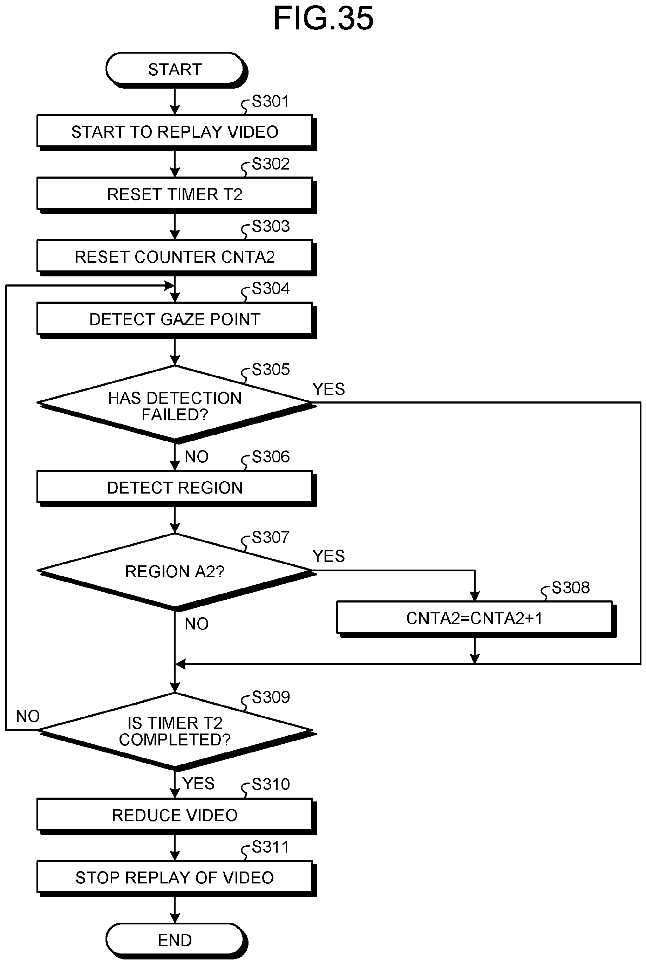

Next, an example of the evaluation method according to the present embodiment will be described with reference to FIG. 34 and FIG. 35. FIG. 34 is a flowchart illustrating another example of a process in the first display operation. First, as illustrated in FIG. 34, in the first display operation, the display control unit 202 starts to replay an evaluation video that includes the question information I5, the apple graphic images FA1, and the lemon graphic images FB1 (Step S201). After a lapse of a waiting time for the start of the evaluation video, the timer T1 is reset (Step S202), and the count value CNTA1 of the counter is reset (Step S203). The timer T1 is a timer for obtaining a timing at which the evaluation video including the question information I5, the apple graphic images FA1, and the lemon graphic images FB1 is terminated. The counter CNTA1 is a device for measuring the count value CNTA1 indicating the first gaze time data. The timer T1 and the counter CNTA1 are arranged in, for example, the arithmetic unit 220.

The gaze point detection unit 214 detects the positional data of the gaze point of the subject on the display screen 101S of the display device 101 with a defined sampling period (for example, 20 msec) while showing the video displayed on the display device 101 to the subject (Step S204). If the positional data is not detected (Yes at Step S205), processes from Step S209 to be described later are performed. If the positional data is detected (No at Step S205), the determination unit 218 determines a region in which the gaze point P is present on the basis of the positional data (Step S206).

If it is determined that the gaze point P is present in the corresponding region A1 (Yes at Step S207), the arithmetic unit 220 increments the count value CNTA1 indicating the first gaze time data in the corresponding region A1 by 1 (Step S208). Thereafter, the arithmetic unit 220 performs processes from Step S209 to be described later. If it is determined that the gaze point P is not present in the corresponding region A1 (No at Step S207), the processes from Step S209 are performed.

Thereafter, the arithmetic unit 220 determines whether a time at which replay of the video is completed has come, on the basis of a detection result of the timer T1 (Step S209). If the arithmetic unit 220 determines that the time at which replay of the video is completed has not yet come (No at Step S209), the arithmetic unit 220 repeats the processes from Step S204 as described above.

If the arithmetic unit 220 determines that the time at which replay of the video is completed has come (Yes at Step S209), the display control unit 202 stops replay of the video (Step S210). After replay of the video is stopped, operation of displaying the question information I6 or the like is performed.

FIG. 35 is a flowchart illustrating another example of the process performed in the first display operation and the second display operation. As illustrated in FIG. 35, the display control unit 202 first starts to replay an evaluation video including the question information I6, the banana graphic images FA2, and the strawberry graphic images FB2 (Step S301). After a lapse of a waiting time for the start of the evaluation video, the timer T2 is reset (Step S302), and the count value CNTA2 of the counter is reset (Step S303). The timer T2 is a timer for obtaining a timing at which the evaluation video including the question information I6, the banana graphic images FA2, and the strawberry graphic images FB2 is terminated. The counter CNTA2 is a device for measuring the count value CNTA2 indicating the second gaze time data. The timer T2 and the counter CNTA2 are arranged in, for example, the arithmetic unit 220.

The gaze point detection unit 214 detects the positional data of the gaze point of the subject, similarly to operation of displaying the question information I5 or the like (Step S304), and if the positional data is not detected (Yes at Step S305), the processes from Step S309 to be described later are performed. If the positional data is detected (No at Step S305), the determination unit 218 determines a region in which the gaze point P is present, on the basis of the positional data (Step S306).

If it is determined that the gaze point P is present in the corresponding region A2 (Yes at Step S307), the arithmetic unit 220 increments the count value CNTA2, which indicates the second gaze time data in the corresponding region A2, by 1 (Step S308). Thereafter, the arithmetic unit 220 performs the processes from Step S309 to be described later. If it is determined that the gaze point P is not present in the corresponding region A2 (No at Step S307), the processes from Step S309 to be described later are performed.

Thereafter, the arithmetic unit 220 determines whether a time at which replay of the video is completed has come, on the basis of a detection result of the timer T2 (Step S309). If the arithmetic unit 220 determines that the time at which replay of the video is completed has not yet come (No at Step S309), the arithmetic unit 220 repeats the processes from Step S304 as described above.

If the arithmetic unit 220 determines that the time at which replay of the evaluation video including the question information I6 and the like is completed has come (Yes at Step S309), the display control unit 202 displays a certain part including the question information I7 in the evaluation video on the display screen 101S. After displaying the question information I7 for a predetermined time, the display control unit 202 performs the second display operation by displaying the video of the guidance target object E4 as an eye-catching video (Step S310). After displaying the video of the guidance target object E4, the display control unit 202 stops replay of the video (Step S311).

After Step S311, the display control unit 202 performs the third display operation by displaying an evaluation video including the plurality of answer target objects M21 to M24 on the display screen 101S. After performing the third display operation, the evaluation unit 224 obtains evaluation data. Thereafter, the output control unit 226 outputs the evaluation data. The processes in the third display operation, the process of obtaining the evaluation data, and the process of outputting the evaluation data are the same as Step S101 to Step S133 (see FIG. 23) of the embodiment as described above.

As described above, the display control unit 202 is configured to display a plurality of graphic images in the first display operation, display the first question information for instructing the subject to memorize the number of graphic images that match a predetermined condition among the plurality of graphic images, and display the second question information for instructing the subject to perform a calculation using the number of the graphic images that are memorized based on the first question information, so that it is possible to obtain more objective and correct evaluation in a short time, and it is possible to alleviate the influence of mistakes made by a healthy subject.

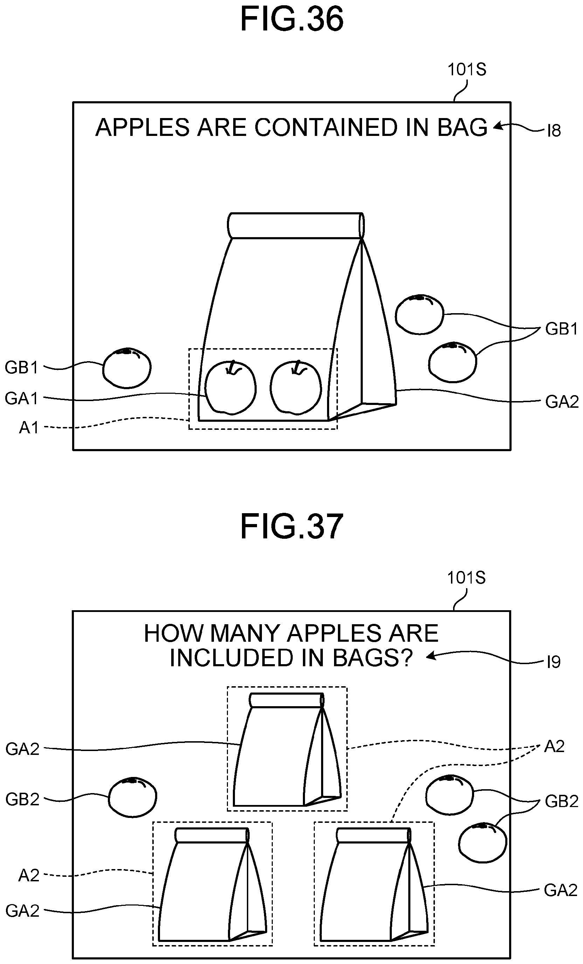

FIG. 36 and FIG. 37 are diagrams illustrating another example of the question information displayed on the display screen 101S. As illustrated in FIG. 36, the display control unit 202 displays instruction information 18, a bag graphic image containing a plurality of apples (the apple graphic images are described as graphic images GA1 and a bag graphic image is described as a graphic image GA2), and a plurality of orange graphic images GB1 on the display screen 101S. The instruction information I8 is an instruction to obtain the number of the apple graphic images GA1 contained in the bag and memorize the number of the apples per bag. Further, the region setting unit 216 sets the corresponding region A1 that corresponds to the apple graphic images GA1. The region setting unit 216 may set the corresponding region A1 in a region that includes at least a part of the apple graphic images GA1. In FIG. 36, the corresponding region A1 is set as a rectangular region including the two apple graphic images GA1, but embodiments are not limited to this example, and the corresponding region A1 may be set for each of the apple graphic images GA1.

The display control unit 202 displays the instruction information 18, the plurality of apple graphic images GA1, the bag graphic image GA2, and the plurality of orange graphic images GB1 as described above on the display screen 101S for a predetermined period. During this period, the gaze point detection unit 214 detects the positional data of the gaze point of the subject on the display screen 101S of the display device 101 with a defined sampling period (for example, 20 msec). If the positional data of the gaze point P is detected, the determination unit 218 determines whether the gaze point of the subject is present in the corresponding region A1 with the above-described sampling period, and outputs determination data. The arithmetic unit 220 calculates the first gaze time data indicating a gaze time for the apple graphic image GA1 indicated by the instruction information I8 on the basis of the determination data.

After displaying the instruction information 18, the plurality of apple graphic images GA1, the bag graphic image GA2, and the plurality of orange graphic patterns GB1 on the display screen 101S for a predetermined period, the display control unit 202 displays question information 19, the plurality of bag graphic images GA2, and the plurality of orange graphic images GB2 on the display screen 101S as illustrated in FIG. 37. The question information I9 is a question indicating an instruction to calculate the number of apple graphic image GA1 contained in the bag (see FIG. 36) on the basis of the number of the bag graphic images GA2. Specifically, a question indicates an instruction to perform multiplication using the number memorized by the subject. Furthermore, the region setting unit 216 sets the corresponding region A2 that corresponds to the bag graphic image GA2. The region setting unit 216 may set the corresponding region A2 in a region that includes at least a part of the bag graphic image GA2.

The display control unit 202 displays the question information 19, the plurality of bag graphic images GA2, and the plurality of orange graphic images GB2 as described above on the display screen 101S for a predetermined period. During this period, the gaze point detection unit 214 detects the positional data of the gaze point of the subject on the display screen 101S of the display device 101 with a defined sampling period (for example, 20 msec). If the positional data of the gaze point P is detected, the determination unit 218 determines whether the gaze point of the subject is present in the corresponding region A2 with the above-described sampling period, and outputs determination data. The arithmetic unit 220 calculates the second gaze time data indicating a gaze time for the bag graphic image GA2 indicated by the question information 19, on the basis of the determination data.

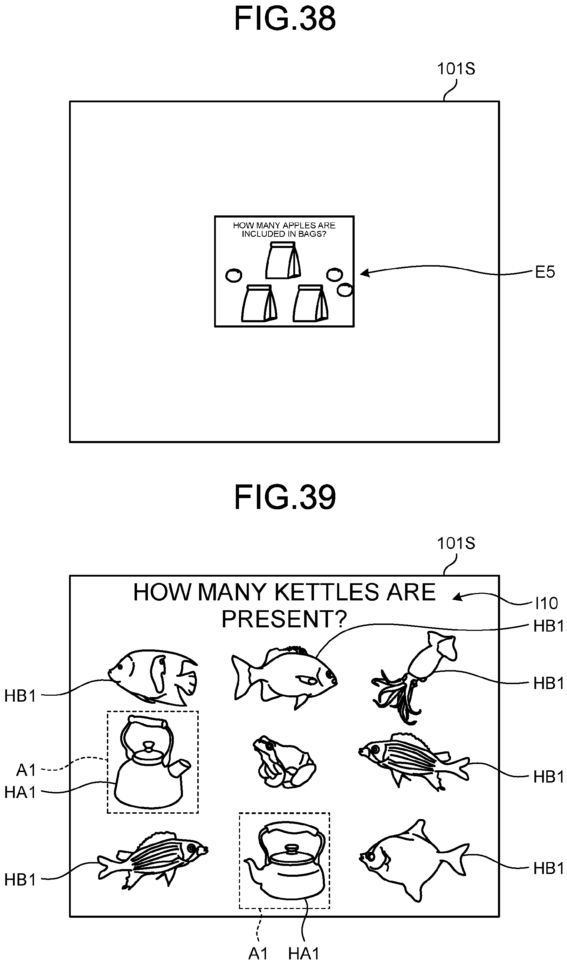

After displaying the question information 19, the plurality of bag graphic images GA2, and the plurality of orange graphic patterns GB2 on the display screen 101S, the display control unit 202 displays, as the second display operation, the guidance target object on the display screen 101S. FIG. 38 is a diagram illustrating another example of a guidance target object displayed on the display screen 101S. As illustrated in FIG. 38, in the second display operation, the display control unit 202 displays a video of the guidance target object E5, which is obtained by reducing the entire image including the question information 19, the plurality of bag graphic images GA2, and the plurality of orange graphic patterns GB2 toward a predetermined target position on the display screen 101S, as an eye-catching video on the display screen 101S. In this example, the position of the center of the display screen 101S is set as the target position, but embodiments are not limited to this example. After performing the second display operation, the display control unit 202 performs the third display operation. The processes from the third display operation are the same as described above.

FIG. 39 to FIG. 41 are diagrams illustrating another example of question information displayed on the display screen 101S. As illustrated in FIG. 39, the display control unit 202 displays question information I10, a plurality of kettle graphic images HA1, and a plurality of creature (fish, a frog, and inkfish) graphic images HB1 on the display screen 101S. The question information I10 is a question indicating an instruction to obtain the number of the kettle graphic images HA1 among the plurality of graphic images and memorize the number. In this example, a larger number of different kinds of graphic images are displayed as compared to the examples as described above, and therefore, the level of difficulty is increased. The question with the increased difficulty as described above is a question used to evaluate a subject who is relatively less likely to have cognitive impairment and brain impairment, and therefore is effective to detect cognitive impairment and brain impairment early, for example. Furthermore, the region setting unit 216 sets the corresponding region A1 that corresponds to the kettle graphic images HA1. The region setting unit 216 may set the corresponding region A1 in a region that includes at least a part of the kettle graphic images HA1.

The display control unit 202 displays the question information I10, the plurality of kettle graphic images HA1, and the plurality of creature graphic images HB1 as described above on the display screen 101S for a predetermined period. During this period, the gaze point detection unit 214 detects the positional data of the gaze point of the subject on the display screen 101S of the display device 101 with a defined sampling period (for example, 20 msec). If the positional data of the gaze point P is detected, the determination unit 218 determines whether the gaze point of the subject is present in the corresponding region A1 with the above-described sampling period, and outputs determination data. The arithmetic unit 220 calculates the first gaze time data indicating a gaze time for the kettle graphic images HA1 indicated by the question information I10, on the basis of the determination data.

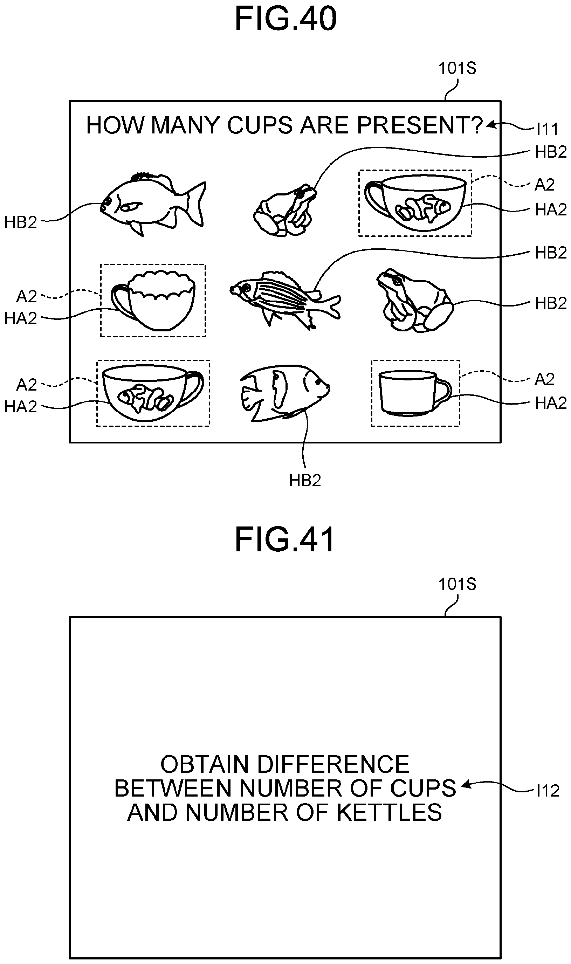

After displaying the question information I10, the plurality of kettle graphic images HA1, and the plurality of creature graphic images HB1 on the display screen 101S for a predetermined period, the display control unit 202 displays the question information I11, a plurality of cup graphic images HA2 and a plurality of creature (fish and frogs) graphic images HB2 on the display screen 101S as illustrated in FIG. 40. The question information I11 is a question indicating an instruction to obtain the number of the cup graphic images among the plurality of graphic images and memorize the number. Further, the region setting unit 216 sets the corresponding region A2 that correspond to the cup graphic images HA2. The region setting unit 216 may set the corresponding region A2 in a region that includes at least a part of the cup graphic images HA2.

The display control unit 202 displays the question information I11, the plurality of cup graphic images HA2, and the plurality of creature graphic images HB2 on the display screen 101S for a predetermined period. During this period, the gaze point detection unit 214 detects the positional data of the gaze point of the subject on the display screen 101S of the display device 101 with a defined sampling period (for example, 20 msec). If the positional data of the gaze point P is detected, the determination unit 218 determines whether the gaze point of the subject is present in the corresponding region A2 with the above-described sampling period, and outputs determination data. The arithmetic unit 220 calculates the second gaze time data indicating a gaze time for the cup graphic images HA2 indicated by the question information I11, on the basis of the determination data.

The question information I11, the plurality of cup graphic images HA2, and the plurality of creature graphic images HB2 are displayed on the display screen 101S for a predetermined period. Thereafter, the display control unit 202 displays, as question information 112, a question instructing the subject to calculate a difference between the number of the cup graphic images HA2 and the number of the kettle graphic images HA1 on the display screen 101S as illustrated in FIG. 41. In this example, a value that is obtained by subtracting the number of the kettle graphic images HA1 that is displayed at an earlier time from the number of the cup graphic images HA2 that is displayed at a later time is to be calculated. Therefore, the level of difficulty increases as compared to the case in which a calculation of subtracting the number of graphic images that is displayed at a later time from the number of graphic images that is displayed at an earlier time.

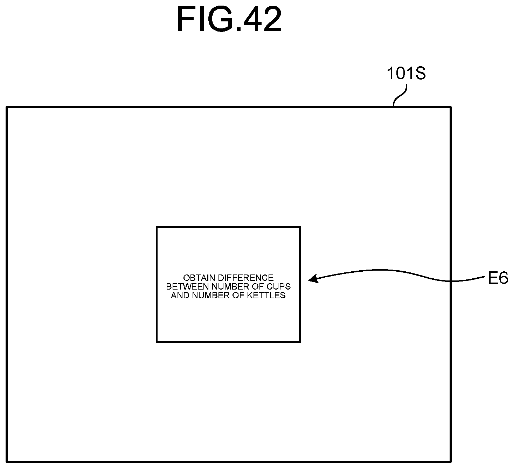

After displaying the question information 112 on the display screen 101S for a predetermined time, the display control unit 202 displays, as the second display operation, the guidance target object on the display screen 101S. FIG. 42 is a diagram illustrating another example of a guidance target object displayed on the display screen 101S. As illustrated in FIG. 42, in the second display operation, the display control unit 202 displays a video of a guidance target object E6, which is obtained by reducing the entire image including the question information 112 toward a predetermined target position on the display screen 101S, as an eye-catching video on the display screen 101S. In this example, the target position is set at the position of the center of the display screen 101S, but embodiments are not limited to this example. The display control unit 202 performs the third display operation after performing the second display operation. The processes from the third display operation are the same as described above.

Reference Signs List

A specific region

A1, A2 corresponding region
B to D comparison region
D1, D2, D3, D4, D5 data value,
E1, E2, E3, E4, E5, E6 guidance target object
FA1, FB1, FA2, FB2, GA1, GB1, GA2, GB2, HA1, HB1, HA2, HB2 graphic image
I1, I2, I4, I5, I6, I7, I9, I10, I11, I12 question information
I3, I8 instruction information
M1, M5, M9, M13, M17 specific target object
M2 to M4, M6 to M8, M10 to M12, M14 to M16, M18 to M20 comparison target object
P gaze point
P1, P1a target position
P2 calculated position
Q region
M1 to M20 answer target object
100 line-of-sight detection apparatus (evaluation apparatus)
101 display device
101S display screen
202 display control unit
214 gaze point detection unit
216 region setting unit
218 determination unit
220 arithmetic unit
224 evaluation unit

**Claims**

1. An evaluation apparatus comprising:

   a display screen (101S);
   a gaze point detection unit (214) configured to detect a position of a gaze point of a subject who observes the display screen (101S);
   a display control unit (202) configured to perform display operation including a first display operation of displaying question information that is a question for the subject on the display screen (101S), a second display operation of displaying a guidance target object that guides the gaze point of the subject to a target position on the display screen (101S), and a third display operation of displaying, on a same circumference of a circle centered at the target position and at positions that do not overlap with the target position, a plurality of answer target objects that are answers for the question on the display screen (101S) after the second display operation;
   a region setting unit (216) configured to set a specific region corresponding to a specific target object among the plurality of the answer target objects and to set comparison regions corresponding to comparison target objects that are different from the specific target object;
   a determination unit (218) configured to determine whether the gaze point is present in the specific region and the comparison regions during the display period in which the third display operation is performed, on the basis of the position of the gaze point;
   an arithmetic unit (220) configured to calculate gaze point data during the display period on the basis of a determination result of the determination unit (218); and
   an evaluation unit (224) configured to obtain evaluation data of the subject on the basis of the gaze point data, wherein the determination unit (218) is configured to detect whether the gaze point is present in a predetermined region including the target position after the second display operation on the basis of the position of the gaze point, and performs the determination if the gaze point is present, or repeats processes from the first display operation if the gaze point is not present.

2. The evaluation apparatus according to claim 1, wherein the display control unit (202) is configured to arrange the plurality of answer target objects at regular pitches on the same circumference of a circle centered at the target position.

3. The evaluation apparatus according to claim 1, wherein the display control unit (202) is configured to arrange and display the plurality of answer target objects on the same circumference of a circle centered at the gaze point of the subject at an end of the second display operation.

4. The evaluation apparatus according to claim 1, wherein the display control unit (202) is configured to display the plurality of answer target objects at regular pitches on the same circumference of a circle centered at the gaze point of the subject at an end of the second display operation.

5. The evaluation apparatus according to any one of claims 1 to 4, wherein the second display operation is configured to reduce at least part of the question information displayed in the first display operation toward the target position.

6. An evaluation method comprising:

displaying an image on a display screen (101S);
detecting a position of a gaze point of a subject who observes the display screen (101S);
performing display operation including a first display operation of displaying question information that is a question for the subject on the display screen (101S), a second display operation of displaying a guidance target object that guides the gaze point of the subject to a target position on the display screen (101S), and a third display operation of displaying, on a same circumference of a circle centered at the target position and at positions that do not overlap with the target position, a plurality of answer target objects that are answers for the question on the display screen (101S) after the second display operation;
setting a specific region corresponding to a specific target object among the plurality of the answer target objects and setting comparison regions corresponding to comparison target objects that are different from the specific target object;
determining whether the gaze point is present in the specific region and the comparison regions during the display period in which
the third display operation is performed, on the basis of the position of the gaze point;
calculating gaze point data during the display period on the basis of a determination result of the determination unit (218); and
obtaining evaluation data of the subject on the basis of the gaze point data,
wherein the determining detects whether the gaze point is present in a predetermined region including the target position after the second display operation on the basis of the position of the gaze point, and performs the determination if the gaze point is present, or repeats processes from the first display operation if the gaze point is not present.

7. An evaluation program that causes a computer to execute:

a process of displaying an image on a display screen (101S);
a process of detecting a position of a gaze point of a subject who observes the display screen (101S);
a process of performing display operation including a first display operation of displaying question information that is a question for the subject on the display screen (101S), a second display operation of displaying a guidance target object that guides the gaze point of the subject to a target position on the display screen (101S), and a third display operation of displaying, on a same circumference of a circle centered at the target position and at positions that do not overlap with the target position, a plurality of answer target objects that are answers for the question on the display screen (101S) after the second display operation;
a process of setting a specific region corresponding to a specific target object among the plurality of the answer target objects and setting
comparison regions corresponding to comparison target objects that are different from the specific target object;
a process of determining whether the gaze point is present in the specific region and the comparison regions during the display
period in which the third display operation is performed, on the basis of the position of the gaze point;
a process of calculating gaze point data during the display period on the basis of a determination result of the determination unit (218); and
a process of obtaining evaluation data of the subject on the basis of the gaze point data,
wherein the determining detects whether the gaze point is present in a predetermined region including the target position after the second display operation on the basis of the position of the gaze point, and performs the determination if the gaze point is present, or repeats processes from the first display operation if the gaze point is not present.

**Patentansprüche**

1.  Eine Auswertungsvorrichtung, umfassend:

    einen Anzeigebildschirm (101S);
    eine Blickpunktdetektionseinheit (214), die konfiguriert ist, um eine Position eines Blickpunkts eines Subjekts zu detektieren, das den Anzeigebildschirm (101S) beobachtet;
    eine Anzeigesteuereinheit (202), die konfiguriert ist, um eine Anzeigeoperation durchzuführen, einschließlich einer ersten Anzeigeoperation des Anzeigens von Frageinformationen, die eine Frage für das Subjekt auf dem Anzeigebildschirm (101S) sind, einer zweiten Anzeigeoperation des Anzeigens eines Führungszielobjekts, das den Blickpunkt des Subjekts zu einer Zielposition auf dem Anzeigebildschirm (101S) führt, und einer dritten Anzeigeoperation des Anzeigens, auf einem gleichen Umfang eines Kreises, der bei der Zielposition zentriert ist, und an Positionen, die nicht mit der Zielposition überlappen, einer Vielzahl von Antwortzielobjekten, die Antworten für die Frage auf dem Anzeigebildschirm (101S) sind, nach der zweiten Anzeigeoperation;
    eine Bereichseinstelleinheit (216), die konfiguriert ist, um einen spezifischen Bereich, der einem spezifischen Zielobjekt entspricht, aus der Vielzahl der Antwortzielobjekte einzustellen und um Vergleichsbereiche einzustellen, die Vergleichszielobjekten entsprechen, die sich von dem spezifischen Zielobjekt unterscheiden;
    eine Bestimmungseinheit (218), die konfiguriert ist, um zu bestimmen, ob der Blickpunkt in dem spezifischen Bereich und den Vergleichsbereichen während der Anzeigeperiode, in der die dritte Anzeigeoperation durchgeführt wird, vorhanden ist, basierend auf der Position des Blickpunkts;
    eine Arithmetikeinheit (220), die konfiguriert ist, um Blickpunktdaten während der Anzeigeperiode zu berechnen, basierend auf einem Ergebnis der Bestimmung der Bestimmungseinheit (218); und
    eine Auswertungseinheit (224), die konfiguriert ist, um Auswertungsdaten des Subjekts zu erhalten, basierend auf den Blickpunktdaten,
    wobei die Bestimmungseinheit (218) konfiguriert ist, um zu detektieren, ob der Blickpunkt in einem vorbestimmten Bereich einschließlich der Zielposition nach der zweiten Anzeigeoperation vorhanden ist, basierend auf der Position des Blickpunkts, und die Bestimmung durchführt, wenn der Blickpunkt vorhanden ist, oder die Prozesse von der ersten Anzeigeoperation wiederholt, wenn der Blickpunkt nicht vorhanden ist.

2.  Auswertungsvorrichtung nach Anspruch 1, wobei die Anzeigesteuereinheit (202) konfiguriert ist, um die Vielzahl von Antwortzielobjekten in regelmäßigen Abständen auf demselben Umfang eines Kreises anzuordnen, der bei der Zielposition zentriert ist.

3.  Auswertungsvorrichtung nach Anspruch 1, wobei die Anzeigesteuereinheit (202) konfiguriert ist, um die Vielzahl von Antwortzielobjekten auf dem gleichen Umfang eines Kreises anzuordnen und anzuzeigen, der an einem Ende der zweiten Anzeigeoperation bei dem Blickpunkt des Subjekts zentriert ist.

4.  Auswertungsvorrichtung nach Anspruch 1, wobei die Anzeigesteuereinheit (202) konfiguriert ist, um die Vielzahl von Antwortzielobjekten in regelmäßigen Abständen auf dem gleichen Umfang eines Kreises anzuzeigen, der an einem Ende der zweiten Anzeigeoperation bei dem Blickpunkt des Subjekts zentriert ist.

5.  Auswertungsvorrichtung nach irgendeinem der Ansprüche 1 bis 4, wobei die zweite Anzeigeoperation konfiguriert ist, um zumindest einen Teil der in der ersten Anzeigeoperation angezeigten Frageinformationen, die in Richtung der Zielposition angezeigt werden, zu reduzieren.

6.  Ein Auswertungsverfahren, umfassend:

    Anzeigen eines Bildes auf einem Anzeigebildschirm (101S);
    Detektieren einer Position eines Blickpunktes eines Subjekts, das den Anzeigebildschirm beobachtet (101S);
    Durchführen einer Anzeigeoperation, einschließlich einer ersten Anzeigeoperation des Anzeigens von Frageinformationen, die eine Frage für das Subjekt auf dem Anzeigebildschirm (101S) sind, einer zweiten Anzeigeoperation des Anzeigens eines Führungszielobjekts, das den Blickpunkt des Subjekts zu einer Zielposition auf dem Anzeigebildschirm (101S) führt, und einer dritten Anzeigeoperation des Anzeigens, auf einem gleichen Umfang eines Kreises, der bei der Zielposition zentriert ist, und an Positionen, die nicht mit der Zielposition überlappen, einer Vielzahl von Antwortzielobjekten, die Antworten für die Frage auf dem Anzeigebildschirm (101S) sind, nach der zweiten Anzeigeoperation;
    Einstellen eines spezifischen Bereichs, der einem spezifischen Zielobjekt aus der Vielzahl von Antwortzielobjekten entspricht, und Einstellen von Vergleichsbereichen, die Vergleichszielobjekten entsprechen, die sich von dem spezifischen Zielobjekt unterscheiden;

Bestimmen, ob der Blickpunkt in dem spezifischen Bereich und den Vergleichsbereichen während der Anzeigeperiode, in der die dritte Anzeigeoperation durchgeführt wird, vorhanden ist, basierend auf der Position des Blickpunkts;

Berechnen von Blickpunktdaten während der Anzeigeperiode, basierend auf einem Ergebnis der Bestimmung der Bestimmungseinheit (218); und

Erhalten von Auswertungsdaten des Subjekts, basierend auf den Blickpunktdaten,

wobei das Bestimmen detektiert, ob der Blickpunkt in einem vorbestimmten Bereich einschließlich der Zielposition nach der zweiten Anzeigeoperation vorhanden ist, basierend auf der Position des Blickpunkts, und das Bestimmen durchführt, wenn der Blickpunkt vorhanden ist, oder die Prozesse von der ersten Anzeigeoperation wiederholt, wenn der Blickpunkt nicht vorhanden ist.

**7.** Ein Auswertungsprogramm, das einen Computer dazu veranlasst, Folgendes auszuführen:

einen Prozess des Anzeigens eines Bildes auf einem Anzeigebildschirm (101S);

einen Prozess des Detektierens einer Position eines Blickpunktes eines Subjekts, das den Anzeigebildschirm beobachtet (101S);

einen Prozess des Durchführens einer Anzeigeoperation, einschließlich einer ersten Anzeigeoperation des Anzeigens von Frageinformationen, die eine Frage für das Subjekt auf dem Anzeigebildschirm (101S) sind, einer zweiten Anzeigeoperation des Anzeigens eines Führungszielobjekts, das den Blickpunkt des Subjekts zu einer Zielposition auf dem Anzeigebildschirm (101S) führt, und einer dritten Anzeigeoperation des Anzeigens, auf einem gleichen Umfang eines Kreises, der bei der Zielposition zentriert ist, und an Positionen, die nicht mit der Zielposition überlappen, einer Vielzahl von Antwortzielobjekten, die Antworten für die Frage auf dem Anzeigebildschirm (101S) sind, nach der zweiten Anzeigeoperation;

Einen Prozess des Einstellens eines spezifischen Bereichs, der einem spezifischen Zielobjekt aus der Vielzahl von Antwortzielobjekten entspricht, und Einstellen von Vergleichsbereichen, die Vergleichszielobjekten entsprechen, die sich von dem spezifischen Zielobjekt unterscheiden;

einen Prozess des Bestimmens, ob der Blickpunkt in dem spezifischen Bereich und den Vergleichsbereichen während der Anzeigeperiode, in der die dritte Anzeigeoperation durchgeführt wird, vorhanden ist, basierend auf der Position des Blickpunkts;

einen Prozess des Berechnens von Blickpunktdaten während der Anzeigeperiode, basierend auf einem Ergebnis der Bestimmung der Bestimmungseinheit (218); und

einen Prozess des Erhaltens von Auswertungsdaten des Subjekts, basierend auf den Blickpunktdaten,

wobei das Bestimmen detektiert, ob der Blickpunkt in einem vorbestimmten Bereich einschließlich der Zielposition nach der zweiten Anzeigeoperation vorhanden ist, basierend auf der Position des Blickpunkts, und das Bestimmen durchführt, wenn der Blickpunkt vorhanden ist, oder die Prozesse von der ersten Anzeigeoperation wiederholt, wenn der Blickpunkt nicht vorhanden ist.

**Revendications**

**1.** Un appareil d'évaluation comprenant :

un écran d'affichage (101S) ;

une unité de détection de point de regard (214) configurée pour détecter une position d'un point de regard d'un sujet qui observe l'écran d'affichage (101S) ;

une unité de commande d'affichage (202) configurée pour effectuer une opération d'affichage comprenant une première opération d'affichage pour afficher des informations de question qui sont une question pour le sujet sur l'écran d'affichage (101S), une deuxième opération d'affichage pour afficher un objet cible de guidage qui guide le point de regard du sujet vers une position cible sur l'écran d'affichage (101S), et une troisième opération d'affichage pour afficher, sur une même circonférence d'un cercle centré sur la position cible et à des positions qui ne se chevauchent pas avec la position cible, une pluralité d'objets cibles de réponse qui sont des réponses à la question sur l'écran d'affichage (101S) après la deuxième opération d'affichage ;

une unité de définition de région (216) configurée pour définir une région spécifique correspondant à un objet cible spécifique parmi la pluralité d'objets cibles de réponse et pour définir des régions de comparaison correspondant à des objets cibles de comparaison qui sont différents de l'objet cible spécifique ;

une unité de détermination (218) configurée pour déterminer si le point de regard est présent dans la région spécifique et les régions de comparaison pendant la période d'affichage dans laquelle la troisième opération d'affichage est effectuée, sur la base de la position du point de regard ;

une unité arithmétique (220) configurée pour calculer des données de point de regard pendant la période d'affichage sur la base d'un résultat de détermination de l'unité de détermination (218) ; et

une unité d'évaluation (224) configurée pour obtenir des données d'évaluation du sujet sur la base des données de point de regard,

dans lequel l'unité de détermination (218) est configurée pour détecter si le point de regard est présent dans une région prédéterminée comprenant la position cible après la seconde opération d'affichage sur la base de la position du point de regard, et effectue la détermination si le point de regard est présent, ou répète les processus de la première opération d'affichage si le point de regard n'est pas présent.

2. Appareil d'évaluation selon la revendication 1, dans lequel l'unité de commande d'affichage (202) est configurée pour disposer la pluralité d'objets cibles de réponse à des pas réguliers sur la même circonférence d'un cercle centré sur la position cible.

3. Appareil d'évaluation selon la revendication 1, dans lequel l'unité de commande d'affichage (202) est configurée pour disposer et afficher la pluralité d'objets cibles de réponse sur la même circonférence d'un cercle centré au point de regard du sujet à une fin de la deuxième opération d'affichage.

4. Appareil d'évaluation selon la revendication 1, dans lequel l'unité de commande d'affichage (202) est configurée pour afficher la pluralité d'objets cibles de réponse à des pas réguliers sur la même circonférence d'un cercle centré au point de regard du sujet à une fin de la deuxième opération d'affichage.

5. Appareil d'évaluation selon l'une quelconque des revendications 1 à 4, dans lequel la deuxième opération d'affichage est configurée pour réduire au moins une partie des informations de la question affichée dans la première opération d'affichage vers la position cible.

6. Procédé d'évaluation comprenant :

afficher une image sur un écran d'affichage (101S) ;

détecter une position d'un point de regard d'un sujet qui observe l'écran d'affichage (101S) ;

effectuer une opération d'affichage comprenant une première opération d'affichage pour afficher des informations de question qui sont une question pour le sujet sur l'écran d'affichage (101S), une deuxième opération d'affichage pour afficher un objet cible de guidage qui guide le point de regard du sujet vers une position cible sur l'écran d'affichage (101S), et une troisième opération d'affichage pour afficher, sur une même circonférence d'un cercle centré sur la position cible et à des positions qui ne se chevauchent pas avec la position cible, une pluralité d'objets cibles de réponse qui sont des réponses à la question sur l'écran d'affichage (101S) après la deuxième opération d'affichage ;

définir une région spécifique correspondant à un objet cible spécifique parmi la pluralité d'objets cibles de réponse et définir des régions de comparaison correspondant à des objets cibles de comparaison qui sont différents de l'objet cible spécifique ;

déterminer si le point de regard est présent dans la région spécifique et les régions de comparaison pendant la période d'affichage dans laquelle la troisième opération d'affichage est effectuée, sur la base de la position du point de regard ;

calculer des données de point de regard pendant la période d'affichage sur la base d'un résultat de détermination de l'unité de détermination (218) ; et

obtenir des données d'évaluation du sujet sur la base des données de point de regard,

dans lequel la détermination détecte si le point de regard est présent dans une région prédéterminée comprenant la position cible après la seconde opération d'affichage sur la base de la position du point de regard, et effectue la détermination si le point de regard est présent, ou répète les processus de la première opération d'affichage si le point de regard n'est pas présent.

7. Un programme d'évaluation qui fait qu'un ordinateur exécute :

un processus pour afficher une image sur un écran d'affichage (101S) ;

un processus pour détecter une position d'un point de regard d'un sujet qui observe l'écran d'affichage (101S) ;

un processus pour effectuer une opération d'affichage comprenant une première opération d'affichage pour afficher des informations de question qui sont une question pour le sujet sur l'écran d'affichage (101S), une deuxième opération d'affichage pour afficher un objet cible de guidage qui guide le point de regard du sujet vers une position cible sur l'écran d'affichage (101S), et une troisième opération d'affichage pour afficher, sur une même circonférence d'un cercle centré sur la position cible et à des positions qui ne se chevauchent pas avec la position cible, une pluralité d'objets cibles de réponse qui sont des réponses à la question sur l'écran d'affichage (101S) après la deuxième opération d'affichage ;

un processus pour définir une région spécifique correspondant à un objet cible spécifique parmi la pluralité d'objets cibles de réponse et définir des régions de comparaison correspondant à des objets cibles de comparaison qui sont différents de l'objet cible spécifique ;

un processus pour déterminer si le point de regard est présent dans la région spécifique et les régions de comparaison pendant la période d'affichage dans laquelle la troisième opération d'affichage est effectuée, sur la base de la position du point de regard ;

un processus pour calculer des données de point de regard pendant la période d'affichage sur la base d'un résultat de détermination de l'unité de détermination (218) ; et

un processus pour obtenir des données d'évaluation du sujet sur la base des données de point de regard,

dans lequel la détermination détecte si le point de regard est présent dans une région prédéterminée comprenant la position cible après la seconde opération d'affichage sur la base de la position du point de regard, et effectue la détermination si le point de regard est présent, ou répète les processus de la première opération d'affichage si le point de regard n'est pas présent.

# FIG.1

# FIG.2

# FIG.3

100

| | | | |
|---|---|---|---|
| **20** | **30** | **40** | |

20
- DISPLAY CONTROL UNIT — 202
- LIGHT SOURCE CONTROL UNIT — 204
- IMAGE DATA ACQUISITION UNIT — 206
- INPUT DATA ACQUISITION UNIT — 208
- POSITION DETECTION UNIT — 210
- CURVATURE CENTER CALCULATION UNIT — 212
- GAZE POINT DETECTION UNIT — 214
- REGION SETTING UNIT — 216
- DETERMINATION UNIT — 218
- ARITHMETIC UNIT — 220
- STORAGE UNIT — 222
- EVALUATION UNIT — 224
- OUTPUT CONTROL UNIT — 226

30
- INPUT-OUTPUT UNIT — 302

40
- DISPLAY DEVICE DRIVING UNIT — 402
- FIRST CAMERA INPUT-OUTPUT UNIT — 404A
- SECOND CAMERA INPUT-OUTPUT UNIT — 404B
- LIGHT SOURCE DRIVING UNIT — 406

- DISPLAY DEVICE — 101
- FIRST CAMERA — 102A
- SECOND CAMERA — 102B
- FIRST LIGHT SOURCE — 103A
- SECOND LIGHT SOURCE — 103B
- 102
- 103
- OUTPUT DEVICE — 50
- INPUT DEVICE — 60

# FIG.4

# FIG.5

# FIG.6

# FIG.7

# FIG.8

101S

DETECT NET OF CUBE
ON NEXT SCREEN.
GAZE AT CORRECT FIGURE

I1a

I1

I1b

# FIG.9

101S

DETECT NET OF CUBE
ON NEXT SCREEN.
GAZE AT CORRECT FIGURE

E1

P1

# FIG.10

# FIG.11

# FIG.12

101S

HOW MANY TRIANGLES ARE
INCLUDED IN FIGURE BELOW?
GAZE AT NUMBER AS ANSWER
ON NEXT SCREEN.

I2a

I2

I2b

# FIG.13

101S

E2

P1

# FIG.14

M8a(M8)    M8c(M8)    M8b(M8)    101S

16

9

10

15    M7a(M7)

11    M7b(M7)

P1

14

13

12

M6a(M6)    M5    M6b(M6)

# FIG.15

D    M8a(M8)    D    M8c(M8)    D    M8b(M8)    101S

16

9

10

C

15

P

11    C

M7a(M7)

M7b(M7)

14

13

12

M6a(M6)    B    M5    A    M6b(M6)    B

# FIG.16

# FIG.17

# FIG.18

101S

MEMORIZE PERSON BELOW

I3a

I3

I3b

# FIG.19

101S

SEARCH FOR SAME PERSON

I4a

I4

I4b

# FIG.20

# FIG.21

# FIG.22

FIG.23

START

START TO REPLAY VIDEO — S101

WAITING TIME FOR EVALUATION VIDEO PART — S102

RESET TIMER T1 — S103

RESET COUNTER CNTA — S104

FLAG F=0 — S105

DETECT GAZE POINT — S106

S107 HAS DETECTION FAILED? — YES

NO

DETECT REGION — S108

S109 REGION A? — YES

NO S118 REGION B? — YES

NO S122 REGION C? — YES

NO S126 REGION D? — NO

YES S127 FINAL REGION =REGION D? — YES

NO

ROUTE NUMBER =ROUTE NUMBER+1 — S128

FINAL REGION=REGION D — S129

S119 FINAL REGION AR= REGION B? — YES

NO S120

ROUTE NUMBER =ROUTE NUMBER+1

S121 FINAL REGION =REGION B

S123 FINAL REGION =REGION C? — YES

NO S124

ROUTE NUMBER =ROUTE NUMBER+1

S125 FINAL REGION =REGION C

S110 FLAG F=1? — YES

NO S111

SEARCH TIME =TIMER T1

S112 ROUTE NUMBER TO REGION A1 =ROUTE NUMBER

S113 FLAG F=1

S114 FINAL REGION AR= REGION A? — YES

NO S115

ROUTE NUMBER =ROUTE NUMBER+1

S116 FINAL REGION =REGION A

S117 CNTA=CNTA+1

S130 IS TIMER T1 COMPLETED? — NO

YES

STOP REPLAY OF VIDEO — S131

CALCULATE EVALUATION — S132

OUTPUT EVALUATION VALUE — S133

END

# FIG.24

DETECT NET OF CUBE
ON NEXT SCREEN.
GAZE AT CORRECT FIGURE

# FIG.25

START

START TO REPLAY VIDEO — S101

WAITING TIME FOR EVALUATION VIDEO PART — S102

DETECT GAZE POINT — S140

HAS DETECTION FAILED? — S141 — YES

NO

DETECT REGION — S142

EYE-CATCHING CENTER REGION? — S143 — NO

STOP REPLY OF VIDEO — S144

YES

RESET TIMER T1 — S103

RESET COUNTER CNTA — S104

FLAG F=0 — S105

DETECT GAZE POINT — S106

HAS DETECTION FAILED? — S107 — YES

NO

DETECT REGION — S108

REGION A? — S109 — YES

NO

REGION B? — S118 — YES

NO

REGION C? — S122 — YES

NO

REGION D? — S126 — NO

YES

FINAL REGION =REGION D? — S127 — YES

NO

ROUTE NUMBER =ROUTE NUMBER+1 — S128

FINAL REGION=REGION D — S129

FINAL REGION AR= REGION B? — S119 — YES

NO

ROUTE NUMBER =ROUTE NUMBER+1 — S120

FINAL REGION =REGION B — S121

FINAL REGION =REGION C? — S123 — YES

NO

ROUTE NUMBER =ROUTE NUMBER+1 — S124

FINAL REGION =REGION C — S125

FLAG F=1? — S110 — YES

NO

SEARCH TIME =TIMER T1 — S111

ROUTE NUMBER TO REGION A1 =ROUTE NUMBER — S112

FLAG F=1 — S113

FINAL REGION AR= REGION A? — S114 — YES

NO

ROUTE NUMBER =ROUTE NUMBER+1 — S115

FINAL REGION =REGION A — S116

CNTA=CNTA+1 — S117

IS TIMER T1 COMPLETED? — S130 — NO

YES

STOP REPLAY OF VIDEO — S131

CALCULATE EVALUATION — S132

OUTPUT EVALUATION VALUE — S133

END

44

# FIG.26

# FIG.27

# FIG.28

```
                    ( START )

            START TO REPLAY VIDEO        ~S101

            WAITING TIME FOR             ~S102
            EVALUATION VIDEO PART

            DETECT GAZE POINT            ~S140

                  S141
            HAS DETECTION          YES
              FAILED?        ──────────┐
                  │ NO                 │
            DETECT POSITION      ~S145 │
                  │                    │
              S146                     │  S144
            ADJUST ARRANGEMENT    STOP REPLY OF VIDEO
                  │
            RESET TIMER T1              ~S103
                  │
            RESET COUNTER CNTA          ~S104
                  │
            FLAG F=0                    ~S105
                  │
            DETECT GAZE POINT           ~S106
                  S107
      YES   HAS DETECTION                              S110
   ┌────── FAILED?                               FLAG F=1?    YES
   │          │ NO                                   │ NO        │
   │    DETECT REGION    ~S108                  SEARCH TIME  ~S111
   │          S109                              =TIMER T1       │
   │      REGION A?   YES                                        │
   │          │ NO                    S119      ROUTE NUMBER  ~S112
   │          S118              FINAL            TO REGION A1
   │      REGION B?   YES    REGION AR=   YES    =ROUTE NUMBER
   │          │ NO           REGION B?              │
   │          S122              │ NO     S120   FLAG F=1     ~S113
   │      REGION C?   YES    ROUTE NUMBER              │
   │          │ NO           =ROUTE NUMBER+1          S114
   │          S126              │          S121    FINAL
 NO│      REGION D?          FINAL REGION         REGION AR=   YES
   │          │ YES          =REGION B            REGION A?       │
   │          S127                                   │ NO     S115
YES│      FINAL REGION    S123                   ROUTE NUMBER
   │      =REGION D?   YES   FINAL REGION         =ROUTE NUMBER+1
   │          │ NO           =REGION C?   YES        │
   │    ROUTE NUMBER            │ NO      S124   FINAL        S116
   │    =ROUTE NUMBER+1  ~S128 ROUTE NUMBER       REGION
   │          │              =ROUTE NUMBER+1     =REGION A
   │    FINAL REGION=REGION D ~S129  │   S125       │         S117
   │          │              FINAL REGION      CNTA=CNTA+1
   │          S130           =REGION C
 NO│      IS TIMER T1
   └────── COMPLETED?
              │ YES
            STOP REPLAY OF VIDEO        ~S131
              │
            CALCULATE EVALUATION        ~S132
              │
            OUTPUT EVALUATION VALUE     ~S133
              │
            ( END )
```

# FIG.29

101S

HOW MANY APPLES
ARE PRESENT?

I5

A1

FA1

FB1

# FIG.30

101S

HOW MANY BANANAS
ARE PRESENT?

I6

A2
FA2

A2
FA2

FB2

# FIG.31

101S

OBTAIN DIFFERENCE
BETWEEN NUMBER OF APPLES
AND NUMBER OF BANANAS

I7

# FIG.32

101S

OBTAIN DIFFERENCE
BETWEEN NUMBER OF APPLES
AND NUMBER OF BANANAS

E4

# FIG.33

# FIG.34

START

S201
START TO REPLAY VIDEO

S202
RESET TIMER T1

S203
RESET COUNTER CNTA1

S204
DETECT GAZE POINT

S205
HAS DETECTION FAILED? —— YES

NO

S206
DETECT REGION

S207
REGION A1? —— YES

S208
CNTA1=CNTA1+1

NO

S209
IS TIMER T1 COMPLETED?

NO

YES

S210
STOP REPLAY OF VIDEO

END

50

# FIG.35

```
              ┌──────────────┐
              │    START     │
              └──────┬───────┘
                     │        ⌇S301
              ┌──────▼───────────┐
              │ START TO REPLAY VIDEO │
              └──────┬───────────┘
                     │        ⌇S302
              ┌──────▼───────────┐
              │  RESET TIMER T2  │
              └──────┬───────────┘
                     │        ⌇S303
              ┌──────▼───────────┐
              │ RESET COUNTER CNTA2 │
              └──────┬───────────┘
                     │        ⌇S304
              ┌──────▼───────────┐
              │ DETECT GAZE POINT │
              └──────┬───────────┘
                     │        ⌇S305
                  ◇────────◇   YES
                 ╱ HAS DETECTION ╲─────────┐
                 ╲   FAILED?     ╱         │
                  ◇────────◇              │
                     │ NO    ⌇S306         │
              ┌──────▼───────────┐         │
              │  DETECT REGION   │         │
              └──────┬───────────┘         │
                     │        ⌇S307        │
                  ◇────────◇   YES         │
                 ╱ REGION A2? ╲───────┐    │
                 ╲           ╱        │    │
                  ◇────────◇    ⌇S308 │    │
                     │ NO      ┌──────▼──────────┐
                     │         │  CNTA2=CNTA2+1  │
                     │         └──────┬──────────┘
                     │◄───────────────┘
                     │        ⌇S309
     NO           ◇────────◇
    ┌────────────╱ IS TIMER T2 ╲
    │            ╲  COMPLETED?  ╱
    │             ◇────────◇
    │                │ YES    ⌇S310
    │         ┌──────▼───────┐
    │         │ REDUCE VIDEO │
    │         └──────┬───────┘
    │                │        ⌇S311
    │         ┌──────▼───────────┐
    │         │ STOP REPLAY OF VIDEO │
    │         └──────┬───────────┘
    │                │
    │         ┌──────▼───────┐
    │         │     END      │
    │         └──────────────┘
```

# FIG.36

101S

APPLES ARE CONTAINED IN BAG ← I8

GB1

GA1

A1

GB1

GA2

# FIG.37

101S

HOW MANY APPLES ARE INCLUDED IN BAGS? ← I9

GA2

GB2

A2

GA2

A2

GB2

GA2

# FIG.38

101S

HOW MANY APPLES ARE
INCLUDED IN BAGS?

E5

# FIG.39

101S

HOW MANY KETTLES ARE
PRESENT?

I10

HB1

HB1

HB1

HB1

A1

HA1

HB1

HB1

HB1

A1    HA1

# FIG.40

101S

HOW MANY CUPS ARE PRESENT? — I11

HB2

A2

HA2

HB2

HB2

HB2

A2
HA2

A2
HA2

A2
HA2

HB2

# FIG.41

101S

OBTAIN DIFFERENCE
BETWEEN NUMBER OF CUPS
AND NUMBER OF KETTLES — I12

# FIG.42

101S

OBTAIN DIFFERENCE
BETWEEN NUMBER OF CUPS
AND NUMBER OF KETTLES

E6

**EP 3 815 621 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2011083403 A
- US 2010092929 A1
- JP 2003038443 A